(19) Europäisches Patentamt
European Patent Office
Office européen des brevets

(11) **EP 3 666 295 B1**

(12) **EUROPEAN PATENT SPECIFICATION**

(45) Date of publication and mention
of the grant of the patent:
**26.01.2022 Bulletin 2022/04**

(51) International Patent Classification (IPC):
***A61L 15/42*** *(2006.01)*        ***A61L 15/44*** *(2006.01)*
***A61L 15/60*** *(2006.01)*

(21) Application number: **19216246.9**

(22) Date of filing: **13.12.2019**

(52) Cooperative Patent Classification (CPC):
**A61L 15/60; A61L 15/42; A61L 15/44**

(54) **RESILIENT WOUND DRESSING**

ELASTISCHER WUNDVERBAND

PANSEMENT RÉSILIENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.12.2018 GB 201820369**

(43) Date of publication of application:
**17.06.2020 Bulletin 2020/25**

(73) Proprietor: **Advanced Medical Solutions Limited
Winsford, Cheshire CW7 3RT (GB)**

(72) Inventors:
• **Hamerslagh, Brian John
Higher Runcorn, Cheshire WA7 4PB (GB)**

• **Bradford, Colin Raymond
Keighley, West Yorkshire BD20 9AZ (GB)**
• **Bugedo-Albizuri, Ander
Winsford, Cheshire CW7 2LF (GB)**

(74) Representative: **Stafford, Jonathan Alan Lewis
Marks & Clerk LLP
1 New York Street
Manchester M1 4HD (GB)**

(56) References cited:
**EP-A2- 0 321 980        WO-A1-99/06077
WO-A1-2010/035017        GB-A- 2 465 015
US-A- 5 662 634        US-A1- 2003 014 031**

EP 3 666 295 B1

## Description

### Field of the Invention

The present invention relates to a resilient wound dressing and absorbent articles for a resilient wound dressing. The present invention also relates to a method of manufacturing a resilient wound dressing as well as method of manufacturing a resilient absorbent article for a wound dressing.

### Background of the Invention

Surgical wound dressings suitable for use in wound healing conventionally utilise absorbent materials capable of absorbing fluid exuding from the wound. Suitable absorbent materials include non-woven materials such as felts, which are typically highly absorbent and easily manufactured.

A disadvantage associated with the use of such non-woven absorbent materials is that they typically possess poor stretching characteristics. In the case of a needled felt, the needling process will for instance tend to produce a non-woven fabric high in tensile strength but exhibiting less desirable stretching and conformability properties.

When a wound dressing is placed on a wound in the vicinity of a moveable body joint, such as in the region of the elbow, knee, ankle or wrist, the movement of the joint can exert stretching or twisting forces on the wound dressing due to movement of the skin in the vicinity of the joint. This can in turn lead to loss of adhesion of the dressing to the skin and in some cases delamination of layers of the dressing, e.g. where the various layers have different resilience properties. It is therefore desirable to provide wound dressings which provide desirable absorbency whilst also providing robust adhesion to the skin and resistance to delamination when the dressing is placed on the skin surface near a moveable joint.

Various approaches have been proposed in the art for providing wound dressings that are both absorbent and stretchable, but these often require one or more of a complex structure, the use of relatively difficult or laborious manufacturing techniques, and the use of manufacturing techniques that are not readily scalable. Moreover, some prior art solutions are only capable of stretching in a single spatial dimension, such as in the longitudinal direction of the wound dressing. For instance, known methods for providing a resilient function to otherwise non-resilient wound dressing articles include stitch bonding flexible yarns through an absorbent layer of the wound dressing, such as described in WO2010/035017A1. This technique is however relatively difficult to implement, and succeeds in providing flexibility only in a direction parallel to the longitudinal axis of the flexible yarns. Movement of body joints can however exert forces on a wound dressing in multiple directions and thus such dressings are not able to accommodate the full range of movement of joints and so may still be relatively more prone to loss of adhesion or delamination.

Wound dressings which obviate or mitigate one or more of the abovementioned disadvantages would therefore be desirable.

### Summary of the Invention

The present inventors have developed a resilient wound dressing which is eminently suitable for dressing wounds, especially surgical wounds, and particularly suitable for dressing surgical wounds located in the region of moveable joints, such as the elbow and knee.

In a first aspect of the present invention there is provided a resilient wound dressing comprising an absorbent layer comprising a non-woven resilient sheet of elastomeric material bonded to a non-woven absorbent material, an elastomeric backing layer and an elastomeric adhesive layer located between the absorbent layer and the backing layer to adhere the absorbent layer to the backing layer, wherein the non-woven resilient sheet and the non-woven absorbent material are bonded by needle bonding.

The resilient wound dressing of the present invention provides a stretchable and flexible absorbent dressing of relatively simple construction which is resistant to delamination or becoming prematurely detached from the skin of the user, even when located in the vicinity of joints. This is provided for by providing an elastomeric backing layer which is bonded to a resilient (i.e. stretchy) absorbent layer by virtue of an elastomeric adhesive. The inventors have observed that the use of an elastomeric adhesive layer (such as a hydrocolloid layer) as the bond between the backing layer and absorbent layer means that the bond between these layers remains intact and resistant to delamination even when the dressing is subject to significant tension and flexion in use. Because the absorbent layer comprises a sheet of elastomeric material bonded to an absorbent non-woven material, the absorbent layer has the benefit of being stretchable in multiple directions simultaneously, and not simply for instance in the longitudinal direction, as is the case with some prior art wound dressings. This versatile and stretchable dressing is thus capable of efficiently absorbing exudate from a wound whilst stretching and flexing multi-directionally in response to joint movements and thus conforming to the anatomy or profile of the joint during use. The simple laminate structure of this resilient wound dressing advantageously allows for its straightforward and efficient manufacture which is eminently suitable for industrial scale production.

### *Absorbent layer*

The absorbent layer comprises a non-woven resilient sheet of elastomeric material bonded to a non-woven absorbent material. It will be understood that the absorbent layer must be able to receive and retain an aqueous fluid (e.g. wound exudate) flowing from a wound during use. The dressings are suitable for any type of surface wound and. For instance, as described herein, the term wound may include abrasions, lacerations, punctures, surgical incisions, avulsions and other such like wounds.

The resilient sheet according to this aspect comprises elastomeric material, may consist substantially of elastomeric material and in some embodiments consists of elastomeric material.

The non-woven resilient sheet and the non-woven absorbent material are bonded by needle bonding.

This is particularly suitable when the elastomeric material is provided in the form of fibres, such as a fibrous mat, e.g. a melt-blown polymer web, as described below. Where the non-woven resilient sheet and the non-woven absorbent material are bonded by needle bonding, fibres in the non-woven absorbent material are physically punched through and entangled within fibres in the non-woven resilient sheet to securely attach the resilient sheet to the absorbent material. This has the advantage in the present invention of providing a strong bond between the non-woven absorbent material and the elastomeric sheet without the need for any additional substances or for modification of the component materials, thus allowing the materials to freely stretch and flex to improve the conformability of the dressing and without negatively impacting the absorbency.

### *Non-woven resilient sheet of elastomeric material*

The non-woven resilient sheet of the resilient wound dressing of the present invention may include any suitable non-woven elastomeric material. By "sheet", it is meant that the material is substantially flat or planar in its geometry. Because the sheet is elastomeric and resilient, it is able to lengthen in the plane of the material by stretching and also return towards its pre-stretched state when the stretching force is removed (e.g. by retraction). It will be understood that the non-woven resilient sheet is capable of multi-directional stretching when a stretching force is applied to the sheet. The non-woven resilient sheet, when stretched from a pre-expanded to an expanded state upon application of a stretching force, has the ability to return towards its pre-expanded state once the stretching force is removed. This may in embodiments mean that the material can return to substantially the same, e.g. the same, pre-stretch dimensions, or at least similar dimensions (e.g. within 10% of the pre-stretch dimensions).

It is preferred that the resilient sheet allow water vapour, and in embodiments water fluid, to pass therethrough. Preferably, therefore, the sheet is porous (e.g. by way of perforations and / or as spaces between fibres). The non-woven resilient sheet described herein may be provided in the form of non-fibrous elastomeric materials, e.g. obtainable via sheet-extrusion or sheet-casting methods known to the person skilled in the art. For example, the non-woven resilient sheet may be provided in the form of a film or foam. Alternatively, the non-woven resilient sheet may be a fibrous material (i.e. containing fibres or materials including fibres), such as a fabric.

In typical embodiments, the non-woven resilient sheet comprises (and in typical embodiments, is) a polymer web, preferably, a melt-blown polymer web. In preferred embodiments the non-woven resilient sheet is provided in the form of a melt-blown polymer web (i.e. comprising, and in preferred embodiments consisting of, elastomeric fibres). Melt-blown non-woven webs are suitably porous, thus allowing moisture and water vapour to be transmitted therethrough, enhancing the breathability of the dressing. Suitably the melt-blown polymer web comprises elastomeric fibres. The multidirectional array of interlocking elastomeric fibres in a melt-blown web advantageously allows for multi-directional stretchability. The fibrous nature of a melt-blown web also lends itself well to needle-bonding, thus providing an easy and secure way to bond the web to the non-woven absorbent material, e.g. when the non-woven absorbent material is a fabric, such as a felt.

In embodiments, the melt-blown polymer web includes polyethylene and/or polyurethane fibres. Preferably, the melt-blown polymer web includes polyurethane fibres.

For the avoidance of doubt, an elastomeric material as used herein refers to a material containing elastomers. Such elastomers may include any suitable polymer having elastic properties, for example, silicones, polyurethanes, polyethylenes or a combination thereof. In embodiments, the elastomeric material includes a polymer having intrinsic elastic properties which are independent of the shape or geometry of the elastomeric material. Polyurethanes are particularly preferred as the elastomeric polymer, such as polyurethanes having a molecular weight of greater than or equal to 2000.

In some embodiments, the non-woven resilient sheet as described herein has a thickness of from about 0.06mm to about 0.18mm, from about 0.08mm to about 0.18mm, from about 0.1mm to about 0.18mm, from about 0.12mm to about 0.18mm, from about 0.06mm to about 0.18mm, from about 0.06mm to about 0.16mm, from about 0.06mm to about 0.14mm or from about 0.06mm to about 0.12mm. Preferably, the non-woven resilient sheet may have a thickness of from 0.08mm to about 0.16mm or from about 0.1mm to about 0.14mm. In embodiments, the non-woven resilient sheet may have a thickness of from about 0.1mm to about 0.14mm, such as 0.12mm.

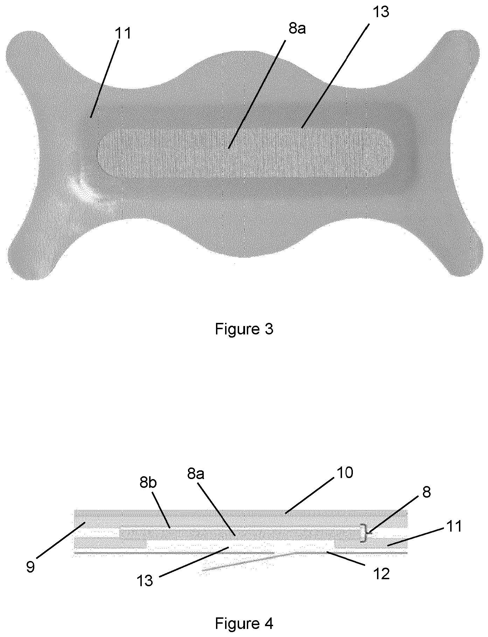

In embodiments, the non-woven resilient sheet of elastomeric material is positioned in the dressing so as to be distal to the wound relative to the absorbent non-woven material, such as depicted as feature 8b in Figure 4.

### *Absorbent non-woven material*

The absorbent non-woven material of the wound dressing of the present invention may include any non-woven material capable of receiving and retaining an aqueous fluid (e.g. wound exudate) flowing from an open wound during use. Such materials may include foams and non-woven fabrics. Preferably the non-woven absorbent material comprises, or in preferred embodiments is, a non-woven fabric. A preferred non-woven fabric is a needled felt. The absorbent non-woven material may be resilient or substantially non-resilient.

In embodiments, non-woven fabrics (e.g. needed felts) may include absorbent fibres. These absorbent fibres may include for example, cellulose fibres (e.g. cotton or viscose), alginate fibres, carboxymethyl cellulose fibres or a blend of any two or more of these fibre types. The fibres may be alginate fibres incorporating (in the alginate fibres) a further polysaccharide (e.g. carboxymethyl cellulose) for the purpose of improving the absorbency of the fibres.

In preferred embodiments, the non-woven material is a non-woven fabric (preferably a needled felt) comprising gelling fibres. It will be understood that gelling fibres gel on absorption of aqueous fluids (e.g. wound exudate). The use of gelling fibres in the absorbent non-woven material of the present invention provides a moist wound healing environment which enhances the wound healing process. Moreover, this property has the advantage that the absorbent non-woven fabric can be used directly as the wound contact surface because the gelling fibres are less prone to adhering to the wound in use. This thus means that that the absorbent layer can advantageously be provided without the need for an additional wound contact layer (e.g. porous layer) between the absorbent layer and wound. Advantageously, the inventors have also observed that the use of gelling fibres provides additional flexibility to the absorbent layer, because the fibres, once gelled in use, are capable of stretching in a way that the dry fibres prior to gelling are not, thus further enhancing the overall resilience of the dressing. In combination with the elastomeric backing layer, elastomeric adhesive layer and elastomeric non-woven sheet, the use of gelling fibres thus provides for addressing that has suitable wound absorbency, advantageous moisture control at the wound and enhanced stretchability.

The gelling fibres according to embodiments of the present invention may include an alginate, carboxymethylcellulose (CMC), carboxymethyl viscose, gelatine, pectin, hydroxyethyl cellulose, hydroxypropyl cellulose, methyl cellulose, sulphonated cellulose (SC), sulphonated viscose, carboxymethyl chitosan, polyvinyl alcohol or any combination thereof. In embodiments, the gelling fibres include an alginate and carboxymethylcellulose (CMC), preferably wherein the alginate and carboxymethylcellulose (CMC) are co-spun to form fibres which each include the alginate and carboxymethylcellulose (CMC). It will be understood that co-spun alginate and carboxymethylcellulose (CMC) gelling fibres are produced from a dope mixture containing both alginate and carboxymethylcellulose (CMC) components. The dope mixture is then subjected to the spinning process in order to provide the co-spun alginate and carboxymethylcellulose (CMC) containing a homogeneous mix of the polymers in the polymer matrix of the gelling fibres. Alternatively, in other embodiments, the gelling fibres may include a mixture of separate alginate and carboxymethylcellulose (CMC) fibres wherein each fibre includes only one of the alginate or carboxymethylcellulose (CMC) components.

The absorbent non-woven material of the wound dressing of the present invention may further include an anti-microbial agent and/or anti-biofilm agent. In embodiments, the anti-microbial agent and/or anti-biofilm agent may be selected from the group consisting of silver, silver compounds (e.g. silver nitrate and silver carbonate), iodine compounds, monoguanides, biguanides (e.g. chlorhexidine and / or Polyhexamethylene biguanide ("PHMB")), cationic surfactants (e.g. octenidine dihydrochloride), biocides, dispersing agents (e.g. cis-2-decenoic acid) or combination thereof. In embodiments, the anti-microbial agent and/or anti-biofilm agent may be a silver compound, preferably silver carbonate.

The gelling fibres according to embodiments of the present invention may include an alginate, carboxymethylcellulose (CMC) and a silver compound, preferably wherein the alginate, carboxymethylcellulose (CMC) and silver compound are co-spun to form fibres which each comprise the alginate, carboxymethylcellulose (CMC) and silver compound. Preferably, the silver compound may be a sparingly soluble particulate inorganic silver salt. In embodiments, the silver compound may be silver carbonate, and particularly particles thereof.

The use of sparingly soluble particulate inorganic silver salts, such as silver carbonate, during the co-spinning process provides absorbent gelling fibres which have advantageous anti-microbial/anti-biofilm effects. This is because during the co-spinning process the particulate inorganic silver salts (e.g. silver carbonate) form discrete sparingly soluble particles dispersed throughout the polymer matrix of the co-spun fibres. During these dispersed particles allow for a slower, controlled, release of silver ions when the fibres come into contact with aqueous fluid (e.g. wound exudate) compared to when salts with a high solubility are used. This is beneficial to the anti-microbial/anti-biofilm effect of the wound dressing during the wound healing process and prevents the amount of silver ion released from being too high, such as when highly soluble silver compounds such as silver nitrate are used, which lead to fast leaching which can in turn have a detrimental impact on wound healing. Conversely, the amount of silver ion being release is also not too low such that antimicrobial/anti-biofilm effects are not sufficient.

In some embodiments, the gelling fibres may include 40-99 wt% of the alginate, 1-60 wt% of the carboxymethylcellulose (CMC) and 0-5 wt% of the silver carbonate relative to the dry mass weight of the gelling fibres. Preferably, the gelling fibres may include 90-95 wt% of the alginate, 1-5 wt% of the carboxymethylcellulose (CMC) and 1-5 wt% of the silver carbonate relative to the dry mass weight of the gelling fibres, even more preferably, 90-95 wt% of the alginate, 3-5 wt% of the carboxymethylcellulose (CMC) and 2-4 wt% of the silver carbonate relative to the dry mass weight of the gelling fibres. It will be appreciated that the relative amounts of the components may vary within the wt% ranges specified above but will not exceed a total of 100 wt% of the gelling fibres when taken in combination.

According to further embodiments, the alginate may have a mannuronic acid : guluronic acid ratio of from about 5 : 95 to about 95 : 5, from about 10 : 90 to about 90 : 10, from about 20 : 80 to about 80: 20, from about 30 : 70 to about 70: 30 or from about 40 : 60 to about 60: 40. In further embodiments, the mannuronic acid : guluronic acid ratio may be about 95: 5, about 90: 10, about 80: 20, about 70: 30, about 60 : 40 or about 50 : 50. Moreover, the alginate may have a mannuronic acid : guluronic acid ratio of from about 90: 10 to about 50 : 50, about 80: 20 to about 50 : 50 or about 70: 30 to about 50 : 50.

In embodiments, the absorbent non-woven material as described herein may have a thickness of from about 2.2mm to about 3.4mm, from about 2.4mm to about 3.4mm, from about 2.6mm to about 3.4mm, from about 2.8mm to about 3.4mm, from about 2.2mm to about 3.2mm, from about 2.2mm to about 3.0mm or from about 2.2mm to about 2.8mm. Preferably, the absorbent non-woven material may have a thickness of from 2.4mm to about 3.2mm or from about 2.6mm to about 3.0mm. In embodiments, the absorbent non-woven material may have a thickness of from about 2.6mm to about 3.0mm.

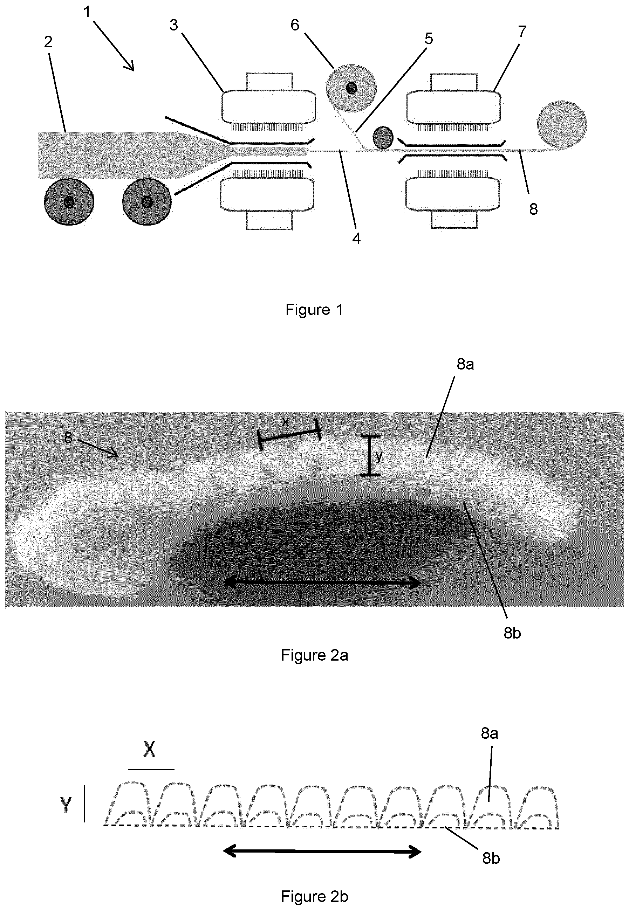

In some embodiments, the non-woven absorbent material may be provided in a fluted configuration (typically when the material is at rest, i.e. not being subject to stretching forces). Put another way, the non-woven absorbent material may be provided in the wound dressing in a corrugated configuration (e.g. wherein the material is gathered to form a number of peaks and troughs). Such a fluted or corrugated configuration is evident in the absorbent material shown in the absorbent layer 8a depicted in Figures 2a and 2b. The inventors have observed that, advantageously, a fluted configuration provides enhanced absorbency to the absorbent layer. This is believed to be caused at least in part by virtue of the channels formed between the periodic corrugations in the fluted configuration aiding in wicking and retaining fluid exuding from the wound. Moreover, the fluting of the non-woven absorbent material allows for more absorbent material to be gathered in a single unit space and thus increases the amount of absorbent material that may be provided in a given unit length of wound dressing relative to when the material is not fluted, thus further increasing the amount of fluid that may be received and retained within the overall resilient wound dressing. In addition, the fluted arrangement is able to flatten (i.e. become substantially planar) and thus lengthen if the wound dressing is stretched during use.

Thus, in advantageous embodiments, the absorbent layer may thus comprise the absorbent non-woven material bonded to the resilient sheet of elastomeric material such that the absorbent layer adopts a fluted configuration when the absorbent layer is not under tension (e.g. in the longitudinal direction) and adopts a substantially flattened (or in other words a substantially non-fluted configuration) when the absorbent layer is placed under tension (e.g. in the longitudinal direction). This in turn may enhance the overall flexibility of the wound dressing of the present invention. This added flexibility in turn is particularly beneficial for use with non-resilient fibres in the non-woven absorbent material as the material can thus lengthen as the dressing is stretched (and thus remain bonded to the resilient non-woven sheet as it stretched) without tearing of the material (e.g. felt), or otherwise degrading the integrity of the absorbent non-woven. This thus enhances the versatility of the dressing because manufacturers in practice have a wider variety of materials to choose from for the absorbent layer without compromising the stretchability / conformability of the dressing.

When the non-woven absorbent material is in a fluted configuration, the average distance between each flute (i.e. distance from peak to peak or trough to trough) of the fluted configuration may be from about 2mm to about 7mm, from about 3mm to about 5mm, from about 3mm to about 6mm, from about 4mm to about 6mm or from about 4mm to about 5mm. Preferably, the average distance between adjacent flutes of the fluted configuration is from about 4mm to about 5mm. It will be appreciated that the distance between adjacent flutes is measured as the distance from the crest (i.e. the top of the flute or corrugation) of a first flute to the crest of an adjacent second flute. This is depicted by distance "x" in Figures 2a and 2b. The average flute height of the fluted configuration may be from about 0.1 mm to about 7.0mm, 0.2mm to about 4mm or from about 0.5mm to about 3.5mm. This is depicted by distance "y" in Figures 2a and 2b.

The fluted configuration may be provided by any suitable method known in the art. Such methods include creping techniques wherein a non-woven absorbent material as described herein undergoes creping (e.g. dry creping) to produce a fluted or corrugated configuration within the non-woven absorbent material. The fluted non-woven absorbent material may then be adhered or bonded to a non-woven resilient sheet of elastomeric material as described herein using needle bonding. Alternatively, in some embodiments, the non-woven absorbent material is gathered into a fluted shape before then bonding the non-woven absorbent material to the non-woven resilient sheet of elastomeric material whilst maintaining the fluted shape to provide the fluted configuration. In embodiments, the gathering is achieved by forming a fluted shape from an elongate length of non-woven absorbent material.

Preferably, however, the fluted configuration of the present invention is not formed using creping techniques. In preferred embodiments, the fluted configuration is achieved by expanding, stretching or tensioning a non-woven resilient sheet of elastomeric material according to the present invention from a pre-expanded state to an expanded state, overlaying the non-woven absorbent material onto the non-woven resilient sheet of elastomeric material in its expanded state before then bonding the non-woven absorbent material to the expanded non-woven resilient sheet of elastomeric material. Bonding is achieved by needle bonding. Once the non-woven absorbent material and expanded non-woven resilient sheet of elastomeric material are bonded, the non-woven resilient sheet of elastomeric material is allowed to return towards the pre-expanded state to provide the fluted configuration. Thus, in embodiments, the absorbent layer is prepared according to the method described above (so as to provide a fluted configuration).

By "expanding", it is meant that the sheet is lengthened (e.g. by stretching) in one or more of the longitudinal and lateral directions relative to the plane of the sheet. Thus, expanding the non-woven resilient sheet may include lengthening in a longitudinal direction and / or a cross direction (i.e. lateral direction) in the plane of the sheet. In advantageous embodiments, the expanding (or stretching) of the non-woven resilient sheet is in both the cross (i.e. lateral) and longitudinal directions relative to the plane of the sheet prior to bonding. Thus, in such embodiments, the pre-expanded state refers to the non-woven resilient sheet of elastomeric material before a stretching force is applied to the sheet.

In advantageous embodiments, the absorbent layer may thus comprise the absorbent non-woven material bonded to the resilient sheet of elastomeric material such that the absorbent layer adopts a fluted configuration when the absorbent layer is not under tension (e.g. in the longitudinal direction) and adopts a substantially flattened (or in other words a substantially non-fluted configuration) when the absorbent layer is placed under tension (e.g. in the longitudinal direction).

In some embodiments, the non-woven absorbent material is positioned so as to be proximal to the wound relative to the non-woven resilient sheet of elastomeric material.

*Elastomeric backing layer*

The elastomeric backing layer of the wound dressing of the present invention may be any material suitable for supporting the absorbent layer as described herein and protecting the resilient wound dressing from the external environment during use (i.e. when attached to the wound). Suitable backing layers include a polyurethane backing film or polyethylene backing film. More preferably, the elastomeric backing layer may be a perforated polyurethane backing film, e.g. a film which allows water vapour to flow through the backing layer from the wound (to benefit breathability) but does not allow liquid water to move through the backing layer in the direction of the wound (to benefit waterproofing).

The elastomeric backing layer is able to stretch when placed under tension and is also capable of returning towards (e.g. substantially or entirely returning to) its original pre-stretched state when the stretching force is removed.

In embodiments, the elastomeric backing layer as described herein may have a thickness of from about $10\mu m$ to about $50\mu m$, from about $10\mu m$ to about $20\mu m$, from about $10\mu m$ to about $18\mu m$, from about $10\mu m$ to about $16\mu m$, from about $12\mu m$ to about $20\mu m$, from about $14\mu m$ to about $20\mu m$, from about $12\mu m$ to about $18\mu m$ or from about $14\mu m$ to about $16\mu m$. Preferably, the elastomeric backing layer may have a thickness of about $10\mu m$, about $11\mu m$, about $12\mu m$, about $13\mu m$, about $14\mu m$, about $15\mu m$, about $16\mu m$, about $17\mu m$, about $18\mu m$, about $19\mu m$ or about $20\mu m$. Preferably, the elastomeric backing layer has a thickness of about $15\mu m$.

*Elastomeric adhesive layer*

The elastomeric adhesive layer of the wound dressing of the present invention may be any elastomeric material suitable for adhering the absorbent layer to the elastomeric backing layer or the resilient wound dressing described herein when located between the absorbent layer and the backing layer. The elastomeric adhesive layer not only acts to secure the absorbent layer to the elastomeric backing layer (i.e. to prevent their detachment or delamination during use) but also, during use, enhances the conformability of the overall resilient wound dressing, particularly, when applied to wounds located in the region of moveable joints.

In further embodiments, there is a further elastomeric adhesive layer located on a proximal surface of the dressing for adhering the proximal surface of the dressing to the skin of a user. As used herein, it is intended that the term "proximal surface" refers to the surface of any material/component/layer described herein which is positioned closest to the wound when the resilient wound dressing is in use. In other words, the wound-facing surface. As used herein, it is intended that the term "distal surface" refers to the surface of any material/component/layer described herein which is positioned furthest from the wound when the resilient wound dressing is in use. In other words, the outward-facing surface. By providing the further elastomeric layer to provide the bond to the skin, this complements the conformability of the dressing and thus prevents delamination, or the peeling of the dressing from the skin in use which may otherwise be caused when less flexible adhesives are provided.

The further elastomeric adhesive layer may, preferably, be windowed (or in other words apertured). When the further elastomeric adhesive layer is windowed, this allows the dressing to be adhered to the skin without the dressing being adhered to the wound. That is, the window of a windowed dressing is intended to encompass the wound such that the adhesive material contacts the skin but not the wound. Suitably, in embodiments, the aperture of the windowed layer allows for the non-woven absorbent material to be in direct contact with the wound (i.e. wherein the aperture of the window is configured to overlay the area of the wound) whilst the dressing is bonded to the skin surrounding the wound. This in turn allows for an increased surface area of the dressing to be available for bonding to the skin of the user whilst still leaving the wound free to interact with the absorbent layer. In such embodiments, the absorbent layer is preferably configured to be in direct contact with the wound in use. That is, in such preferred embodiments, the absorbent layer is provided without an atraumatic adhesive or additional wound contacting layer on the wound facing surface of the absorbent layer. This thus allows for maximised absorbency as there are no additional materials that may otherwise hinder the movement of wound exudate into the wound. This feature, in combination with the combination of elastomeric layers provides for a particularly desirable wound dressing that is simple to manufacture, bonds well with the skin even when applied in the vicinity of highly mobile joints (e.g. the elbow or knee) whilst also increasing absorption of wound exudate to improve wound healing.

In some embodiments, the elastomeric adhesive layer and/or further elastomeric adhesive layer may each independently include a hydrocolloid, silicone adhesive, acrylic adhesive, polyurethane adhesive or any combination thereof. Examples of such materials include hydrocolloids. In such embodiments, the hydrocolloid may include any elastomeric material possessing gel-forming and/or adhesive properties.

Hydrocolloids for use according to the present invention typically include a number of components. The hydrocolloid may typically include a blend of water soluble polymers (e.g. carboxymethylcellulose and/or pectin) together with suitable elastomers and/or adhesives. In some embodiments, the hydrocolloid may include carboxymethylcellulose, polysaccharides and pectin. Moreover, the hydrocolloid of the present invention may include any one of the ingredients specified in the embodiments described below, Table 2 or a combination thereof in a suitable amount.

In embodiments, the hydrocolloid may include a polybutylene, for example, polyisobutylene. The polybutylene may have a wt% (i.e. wt% of the overall hydrocolloid composition) of from about 20 wt% to about 30 wt% or from about 25 wt% to about 30 wt%. In particular, the hydrocolloid may include polyisobutylene having a wt% of from about 20 wt% to about 30 wt% or from about 25 wt% to about 30 wt% relative to the weight of the hydrocolloid composition. The polyisobutylene may be provided in the hydrocolloid in an amount of approximately 28 wt% relative to the weight of the hydrocolloid composition.

In embodiments, the hydrocolloid may include water soluble polymers, for example, carboxymethylcellulose and/or pectin. The hydrocolloid may include such water soluble polymers in a wt% (relative to the weight of the overall hydrocolloid composition) of from about 5 wt% to about 40 wt%, from about 10 wt% to about 40 wt%, from about 10 wt% to about 40 wt% or from about 30 wt% to about 40 wt%. In some embodiments, the hydrocolloid may include carboxymethylcellulose in an amount of from 20 wt% to about 40 wt% or from 30 wt% to about 40 wt%. In addition (or in the alternative), the hydrocolloid may include pectin at from about from about 5 wt% to about 10 wt%. Typically, the hydrocolloid may include carboxymethylcellulose at from 20 wt% to about 40 wt% and pectin at from about 5 wt% to about 10 wt%. Preferably, the hydrocolloid may include carboxymethylcellulose in an amount of from 30 wt% to about 40 wt% and pectin in an amount of from about 5 wt% to about 10 wt%. In one embodiment, the hydrocolloid may include carboxymethylcellulose at approximately 30 wt% and/or pectin at approximately 6 wt%.

In some embodiments, the hydrocolloid may include styrene based block copolymers, for example, poly(styrene-butadiene-styrene) block copolymers, poly(styrene isoprenestyrene) block copolymers or poly(styrene-ethylene-butylene-styrene) block copolymers. The hydrocolloid may include such block copolymers having a wt% (i.e. wt% of the overall hydrocolloid composition) of from about 10 wt% to about 20 wt%, from about 15 wt% to about 20 wt%. Typically, the styrene based block copolymer may be a poly(styrene-butadiene-styrene) block copolymers at from about 15 wt% to about 20 wt%.

The hydrocolloid may also include a hydrocarbon resin, for instance, hydrocarbon resins comprising low molecular weight hydrocarbon polymers, for example Escorez™ type resins. The hydrocolloid may include such hydrocarbon resins having a wt% (i.e. relative to the weight of the overall hydrocolloid composition) of from 5 wt% to about 20 wt%, from 10 wt% to about 20 wt% or from 5 wt% to about 15 wt%. Typically, the hydrocarbon resin is provided in the hydrocolloid an amount of approximately 10 wt% relative to the weight of the overall hydrocolloid composition.

The hydrocolloid may also include a flocking powder, for instance, purified powered cellulose. The hydrocolloid may include such flocking materials having a wt% (i.e. relative to the overall hydrocolloid composition) of from about 5 wt% to about 20 wt%, from 10 wt% to about 20 wt% or from 5 wt% to about 15 wt%. Typically, the flocking powder is approximately 8 wt% relative to the weight of the overall hydrocolloid composition.

The hydrocolloid may also include a preservative having a wt% (i.e. relative to the overall hydrocolloid composition) of from about 0.1 wt% to about 2 wt% or from 0.1 wt% to about 1 wt%. In some embodiments, hydrocolloid may include a mineral oil having a wt% (i.e. relative to the overall hydrocolloid composition) of from 0.01 wt% to about 1 wt%, from about 0.01 wt% to about 0.5 wt%, from about 0.01 wt% to about 0.1 wt%, or from about 0.01 wt% to about 0.05 wt% relative to the weight of the overall hydrocolloid composition.

In some embodiments, the hydrocolloid may include polyisobutylene at from about 25 wt% to about 30 wt%, carboxymethylcellulose at from 30 wt% to about 40 wt%, pectin at from about 5 wt% to about 10 wt%, poly(styrene-butadiene-styrene) block copolymers at from about 15 wt% to about 20 wt%, a hydrocarbon resin at from 5 wt% to about 15 wt%, purified powered cellulose from 5 wt% to about 15 wt%, a preservative from 0.1 wt% to about 1 wt% and/or a mineral oil from about 0.01 wt% to about 0.05 wt% relative to the weight of the overall hydrocolloid composition. It will be appreciated that the relative amounts of the components may vary within the wt% ranges specified above but will not exceed a total of 100 wt% of the overall hydrocolloid when taken in combination.

In other embodiments, the elastomeric adhesive layer and/or further elastomeric adhesive layer may include an elastomeric substrate layer coated with a suitable adhesive. For example, when the elastomeric adhesive layer according to the present invention includes an elastomeric substrate layer coated with a suitable adhesive, the proximal surface of the elastomeric substrate layer adheres via the adhesive to the absorbent layer whereas the distal surface of the elastomeric substrate layer adheres via the adhesive to the backing layer. Here, the use of the elastomeric substrate layer prevents detachment or delamination, during use, of the absorbent layer from the elastomeric backing layer. When the further elastomeric adhesive layer according to the present invention includes an elastomeric substrate layer coated with a suitable adhesive, the proximal surface of the elastomeric substrate layer adheres via the adhesive to the skin of the user whereas the distal surface of the elastomeric substrate layer adheres via the adhesive to the absorbent layer. In such embodiments, suitable elastomeric substrate layers may include foam or films. For example, the elastomeric substrate layer may include a polyurethane film or foam. Suitable adhesives may include a hydrocolloid, silicone adhesive, acrylic adhesive, polyurethane adhesive or any combination thereof. Typically however, the elastomeric adhesive layer does not include an elastomeric substrate layer coated with a suitable adhesive and is preferably provided instead as an inherently adhesive material. Preferred materials for the adhesive layer include the use of a hydrocolloid as described above.

In embodiments, the thickness of the elastomeric adhesive layer of the resilient wound dressing of the present invention is from about 0.1 mm to about 1mm, from about 0.2mm to about 0.8mm, from about 0.2mm to about 0.6mm or from about 0.4mm to about 0.6mm or from about 0.3mm to about 0.5mm. In other embodiments, the thickness of the elastomeric adhesive layer of the resilient wound dressing of the present invention is about 0.2mm, about 0.3mm, about 0.4mm, about 0.5mm or about 0.6mm. Preferably, the thickness of the elastomeric adhesive layer of the resilient wound dressing of the present invention is about 0.3mm to about 0.5mm or typically about 0.4mm.

In embodiments, the thickness of the further elastomeric adhesive layer of the resilient wound dressing of the present invention is from about 0.5mm to about 1.5mm, is from about 0.2mm to about 1.4mm, is from about 0.4mm to about 1.2mm or is from about 0.6mm to about 1.0mm. In some embodiments, the thickness of the further elastomeric adhesive layer of the resilient wound dressing of the present invention is about 0.6mm, about 0.7mm, about 0.8mm, about 0.9mm or about 1.0mm. Preferably, the thickness of the further elastomeric adhesive layer of the resilient wound dressing of the present invention is from about 0.6mm to about 1.0mm or typically about 0.8mm.

### Overall thickness of the resilient wound dressing

The overall thickness of the resilient wound dressing of the present invention may in embodiments be from about 0.5mm to about 10mm, from about 1mm to about 10mm, from about 2mm to about 8mm, from about 4mm to about 6mm or from about 3mm to about 5mm. In some embodiments, the overall thickness of the resilient wound dressing of the present invention is about 0.5mm, 1mm, 2mm, 3mm, 4mm, 5mm, 6mm, 7mm or 8mm. Preferably, the overall thickness of the resilient wound dressing of the present invention from about 3mm to about 5mm or about 4mm.

### Performance characteristics

The resilient wound dressing as described herein may have an absorbency free swell of from about 20 g/100cm$^2$ to about 40 g/100cm$^2$, from about 25 g/100cm$^2$ to about 35 g/100cm$^2$, or from about 30 g/100cm$^2$ to about 35 g/100cm$^2$. Typically, the absorbency free swell of the resilient wound dressing is about 30 g/100cm$^2$, about 31 g/100cm$^2$, about 32 g/100cm$^2$, about 33 g/100cm$^2$, about 34 g/100cm$^2$, or about 30 g/100cm$^2$.

Preferably, the resilient wound dressing as described herein may have an absorbency free swell of from 30 g/100cm$^2$ to about 35 g/100cm$^2$, e.g. 34 g/100cm$^2$.

The resilient wound dressing as described herein may have a retention under load of from about 10 g/100cm$^2$ to about 40 g/100cm$^2$, from about 15 g/100cm$^2$ to about 35 g/100cm$^2$, or from about 20 g/100cm$^2$ to about 30 g/100cm$^2$.

Typically, the absorbency free swell of the resilient wound dressing is about 22 g/100cm$^2$, about 23 g/100cm$^2$, about 24 g/100cm$^2$, about 25 g/100cm$^2$, about 26 g/100cm$^2$, or about 27 g/100cm$^2$. Preferably, the resilient wound dressing as described herein may have a retention under load of from about 20 g/100cm$^2$ to about 30 g/100cm$^2$, e.g. 25 g/100cm$^2$.

In embodiments, resilient wound dressing as described herein may have a moisture vapour transmission rate of at least about 500 g/m$^2$/24hr, or at least about 1000 g/m$^2$/24hr, or at least about 2000 g/m$^2$/24hr. The resilient wound dressing may have a moisture vapour transmission rate of from about 1000 g/m$^2$/24hr to about 4000 g/m$^2$/24hr, or from about 1500 g/m$^2$/24hr to about 3000 g/m$^2$/24hr, or from about 2000 g/m$^2$/24hr to about 2500 g/m$^2$/24hr, or from about 2200 g/m$^2$/24hr to about 2300 g/m$^2$/24hr. Preferably, the moisture vapour transmission rate of the resilient wound dressing is from about 2200 g/m$^2$/24hr to about 2300 g/m$^2$/24hr.

The total fluid handling the resilient wound dressing may be from about 4 grams/10cm$^2$/24 hours to about 12 grams/10cm$^2$/24 hours, preferably, from about 6 grams/10cm$^2$/24 hours to about 10 grams/10cm$^2$/24 hours.

In embodiments resilient wound dressing has an extensibility of from about 1 N/cm to about 10 N/cm, from about 1 N/cm to about 5 N/cm, from about 1 N/cm to about 3, preferably from about 2 N/cm to about 4 N/cm.

The resilient wound dressing of the present invention may have a permanent set (%) value of less than or equal to about 20%, or less than or equal to about 10%, or less than or equal to about 5%, or less than or equal to about 2%, or less than or equal to about 1.5%, or preferably, less than or equal to about 1.25%.

In yet further embodiments, the peel adhesive force of the resilient wound dressing may be from about 10 N/2.5cm to about 20 N/2.5cm, or from about 12 N/2.5cm to about 18 N/2.5cm, or from about 14 N/2.5cm to about 18 N/2.5cm, or preferably from about 15 N/2.5cm to about 16 N/2.5cm.

The resilient wound dressing of the present invention may have a coefficient of friction of less than or equal to 10 N, of less than or equal to 5 N, of less than or equal to 2 N or, preferably, of less than or equal to 1.5 N.

The resilient wound dressing of the present invention may have a bending length of from about 10 mm to about 30mm, from about 15 mm to about 25mm or, preferably about 20mm.

In a second aspect of the present invention there is provided a method of manufacturing a resilient wound dressing according to the first aspect of the present invention or any embodiments thereof including the steps of:

(a)(i) providing a non-woven resilient sheet as described according to any embodiment of the first aspect of the invention, providing a non-woven absorbent material as defined according to any embodiment of the first aspect of the invention, and providing an elastomeric backing layer according to any embodiment of the first aspect of the invention, and

(a)(ii) bonding the non-woven resilient sheet to the non-woven absorbent material to form the absorbent layer, wherein the bonding comprises needle bonding; and

(b) adhering the distal surface of the absorbent layer to the elastomeric backing layer using the elastomeric adhesive layer.

In embodiments of the second aspect, the bonding step (a)(ii) may further include:

expanding the non-woven resilient sheet of elastomeric material from a pre-expanded state to an expanded state; and

bonding the non-woven absorbent material to the expanded non-woven resilient sheet by needle bonding; and

allowing the non-woven resilient sheet to return from the expanded state towards the pre-expanded state to provide the absorbent layer.

By "expanding", it is meant that the sheet is lengthened (e.g. by stretching) in one or more of the longitudinal and lateral directions relative to the plane of the sheet. Thus, expanding the non-woven resilient sheet may be performed in a longitudinal direction and / or a cross direction (i.e. lateral direction) in the plane of the sheet. In advantageous embodiments, the expanding (or stretching) of the non-woven resilient sheet is in both the cross (i.e. lateral) and longitudinal directions relative to the plane of the sheet prior to bonding. The stretching of the resilient sheet may in some examples be performed only in the longitudinal direction.

In a third aspect of the present invention there is provided a method of manufacturing a resilient absorbent article for a wound dressing including the steps of:

(a) expanding a non-woven resilient sheet of elastomeric material from a pre-expanded state to an expanded state; and

(b) bonding a non-woven absorbent material to the expanded non-woven resilient sheet by needle bonding; and

(c) allowing the non-woven resilient sheet to return from the expanded state towards the pre-expanded state to provide the resilient absorbent article.

Resilient absorbent articles as prepared by the third aspect of the invention are suitably useful as the absorbent layer according to embodiments of the first aspect of the invention.

By first expanding (i.e. stretching, the non-woven resilient sheet before bonding the non-woven absorbent material and then allowing the resilient sheet to return towards its pre-expanded state), the non-woven absorbent material need not be inherently resilient itself. In practice, with the bonding being performed by needle bonding, the force of the non-woven resilient sheet returning to its pre-expended state can in embodiments gather the non-woven absorbent material bonded to it, preferably into a fluted / corrugated configuration. Thus, in embodiments, the bonding is performed such that when the resilient sheet is allowed to return from the expanded state to the non-expanded state, the absorbent material forms a fluted (or corrugated) configuration. Because of this gathering effect (e.g. fluting), if the absorbent article is subject to stretching forces in use, the non-woven resilient sheet will be able to lengthen (by flattening) to accommodate the stretch, but without tearing the non-woven absorbent layer as the non-woven absorbent layer will have some slack to allow it to flatten when the resilient sheet is stretched. This thus provides a convenient method of preparing stretchy and conformable absorbent articles for use as absorbent layers for wound dressings which are stretchable in multiple directions (not simply in the longitudinal direction as with some prior art dressings) without requiring the absorbent material to be inherently stretchy. This method thus avoids the need to use tricky techniques such as stitch bonding techniques using flexible yarns or the like to provide a stretch function (such as described in WO2010/035017A1) which are typically difficult to implement, provide flexibility only in a direction parallel to the longitudinal axis of the flexible yarns and are less amenable to high volume production.

In embodiments of the third aspect of the present invention, the method further includes adhering the absorbent article to an elastomeric backing layer using an elastomeric adhesive layer. In this embodiment of the third invention and in the method of the second aspect of the invention described above, the adhering step is most conveniently performed by first adhering the elastomeric adhesive layer to the backing layer to form a layered material comprising 2 layers (backing and adhesive) before then applying the absorbent article to the exposed face of the elastomeric adhesive layer. However, it would be appreciated that this bonding may alternatively be performed by first bonding the elastomeric adhesive to the absorbent article before then bonding the remaining exposed face of the elastomeric adhesive layer to the elastomeric backing layer (i.e. to the proximal surface of the backing layer.

In the second and third aspects of the present invention, the bonding may include needle bonding the non-woven resilient sheet to the non-woven absorbent material to form the absorbent layer.

In further embodiments of the second and third aspects, following the bonding of the non-woven resilient sheet to the non-woven absorbent material, the non-woven absorbent material may adopt a fluted configuration when the resilient sheet is allowed to return to towards the pre-expanded configuration.

In embodiments of the second and third aspects, the methods may further comprise applying a windowed elastomeric adhesive layer to a proximal surface of the absorbent layer or absorbent article (for bonding the wound dressing or article to the skin of a user).

It will be appreciated that the embodiments as described above in relation to the resilient wound dressing of the first aspect of the present invention also apply to the second and third aspects of the present invention. For instance, the absorbent layer, non-woven resilient sheet, non-woven absorbent material, elastomeric backing layer, elastomeric adhesive layer (including the further elastomeric adhesive layer) as described herein with respect to the first aspect of the present invention are intended to also be applicable to the second and third aspects of the present invention. Any embodiments or combination of embodiments of those respective features of the wound dressing of the first aspect of the invention are thus contemplated as embodiments of the corresponding features of the methods of manufacture as described in the second and third aspects of the invention.

For example, the non-woven resilient sheet according to the second and third aspects of the present invention may include a melt-blown polymer web. In such embodiments, the melt-blown polymer web may include polyurethane fibres.

The absorbent non-woven material according to second and third aspects of the present invention may be a non-woven absorbent fabric, preferably wherein the fabric comprises gelling fibres. In embodiments, the gelling fibres may include an alginate, carboxymethylcellulose (CMC), carboxymethyl viscose, gelatine, pectin, hydroxyethyl cellulose, hydroxypropyl cellulose, methyl cellulose, sulphonated cellulose (SC), sulphonated viscose, carboxymethyl chitosan, polyvinyl alcohol or any combination thereof. In further embodiments, the gelling fibres may include an alginate and carboxymethylcellulose (CMC), preferably wherein the alginate and carboxymethylcellulose (CMC) are co-spun to form fibres which each comprise the alginate and carboxymethylcellulose (CMC).

The non-woven absorbent material according to second and third aspects of the present invention may further include an anti-microbial agent and/or anti-biofilm agent. In embodiments, the anti-microbial agent and/or anti-biofilm agent may be selected from the group consisting of silver, silver compounds, iodine compounds, monoguanides, biguanides, cationic surfactants, biocides, dispersing agents or combination thereof. The anti-microbial agent and/or anti-biofilm agent may be a silver compound, preferably silver carbonate.

In embodiments of the second and third aspects of the present invention the gelling fibres may include an alginate, carboxymethylcellulose (CMC) and a silver compound, preferably wherein the alginate, carboxymethylcellulose (CMC) and silver compound are co-spun together to form fibres which each comprise the alginate, carboxymethylcellulose (CMC) and the silver compound.

In further embodiments of the second and third aspects of the present invention, the elastomeric backing layer may be a polyurethane backing film, preferably a perforated polyurethane film.

In a fourth aspect of the present invention there is provided a resilient absorbent article obtained or obtainable according to a method according to the third aspect of the present invention and any embodiments thereof as described herein.

In a fifth aspect of the present invention there is provided a resilient absorbent article (e.g. suitable for a wound dressing) comprising (optionally consisting of) an absorbent non-woven material bonded to a resilient sheet of elastomeric material such that the absorbent layer adopts a fluted configuration when the absorbent article is not under tension (e.g. in the longitudinal direction) and is able to adopt a substantially flattened configuration (or in other words a substantially non-fluted configuration) when the absorbent article is under tension (e.g. in the longitudinal direction). Thus, in use, the absorbent article adopts a fluted configuration when in a pre-expanded state and substantially planar configuration when in an expanded state.

In this aspect, it will be appreciated that the bonding (e.g. needle bonding) of the absorbent non-woven material to the resilient sheet of elastomeric material is conducted in a way so as to cause / allow the absorbent layer to adopt a fluted configuration when the absorbent layer retracts from the expanded state to the pre-expanded state. The skilled person will understand, on reading this application, that this can be achieved in practice by controlling the pattern and location of the needle bonding.

It is intended that the components and properties of the resilient absorbent article of the fifth aspect of the invention may be as described herein in relation to the absorbent layer of the first aspect of the invention. For instance, the absorbent non-woven material may be as described herein in respect of the absorbent non-woven material of the first aspect of the invention and any embodiment thereof. Likewise, the resilient sheet of elastomeric material may be as described herein in respect of the resilient sheet of elastomeric material of the first aspect of the invention and any embodiment thereof. The descriptions of the bonding of the absorbent non-woven article to the resilient sheet as described in relation to the first aspect of the invention may likewise apply to the absorbent article of the fifth aspect of the invention, provided the absorbent material is provided in a fluted configuration as described herein. Any embodiments or combination of embodiments of those respective features of the wound dressing of the first aspect of the invention are thus contemplated as embodiments of the corresponding features of the fifth aspect of the invention.

In a preferred embodiment of the fifth aspect of the invention, the absorbent non-woven material is a needle bonded felt comprising gelling fibres (preferably wherein the fibres comprise alginate, carboxymethylcellulose (CMC) and particles of silver carbonate co-spun together to form fibres which each comprise the alginate, carboxymethylcellulose (CMC) and silver carbonate particles), the resilient sheet is a melt-blown polymer web including elastomeric polyurethane fibres and the absorbent non-woven material is needle bonded to the resilient sheet. The absorbent article may for instance be substantially as described herein in relation to figure 4.

The absorbent article is suitable for absorbing aqueous fluid such as bodily fluid, e.g. wound exudate, and thus may be suitable for use as, or in wound dressings, or for other absorbent uses such as in nappies or incontinence articles where the dressing may be subject to stretching forces in use due to movement of the user's body. The absorbent article may thus be a wound dressing or a component of a wound dressing, or an incontinence article or component of an incontinence article, or a nappy, or component of a nappy. The absorbent article is particularly suitable for use in wound dressings and as such may typically be provided as a wound dressing, or as an absorbent component of a wound dressing, preferably a wound dressing as described herein according to the first aspect of the invention.

In a particularly preferred aspect of the present invention there is provided a resilient wound dressing comprising an absorbent layer, an elastomeric polyurethane backing film; and an elastomeric hydrocolloid adhesive layer located between the absorbent layer and the backing layer to adhere the absorbent layer to the backing layer, the absorbent layer comprising (preferably consisting of) a melt-bonded sheet (i.e. a web) of elastomeric material (preferably an elastomeric polyurethane) needle bonded to an absorbent needled felt comprising (preferably consisting of) gelling fibres (preferably wherein the fibres comprise alginate, carboxymethylcellulose (CMC) and particles of silver carbonate co-spun together to form fibres which each comprise the alginate, carboxymethylcellulose (CMC) and silver carbonate particles). Preferably the elastomeric backing layer is porous (e.g. perforated). Preferably the absorbent layer is orientated in the dressing such that the resilient melt-bonded elastomeric sheet is positioned distal to the wound and the absorbent needle felt is positioned proximal to the wound when in use and even more preferably is configured such that the absorbent needled felt is able to be in direct contact with the wound in use. In embodiments, the non-woven resilient sheet has a thickness of from about 0.1 mm to about 0.14mm, such as 0.12mm.

More particularly, the resilient wound dressing may comprise an absorbent layer, an elastomeric porous polyurethane backing film; and an elastomeric hydrocolloid adhesive layer located between the absorbent layer and the backing film to adhere the absorbent layer to the backing film, the absorbent layer consisting of a melt-bonded web of an elastomeric polyurethane needle bonded to an absorbent needled felt comprising (preferably consisting of) gelling fibres, wherein the fibres comprise alginate, carboxymethylcellulose (CMC) and particles of silver carbonate co-spun together to form fibres which each comprise the alginate, carboxymethylcellulose (CMC) and silver carbonate particles), wherein the absorbent layer is orientated in the dressing such that the resilient melt-bonded elastomeric web is positioned distal to the wound when in use relative to the absorbent needle felt which is positioned relatively more proximal to the wound (i.e. in a mutually stacked configuration) when in use and the dressing is configured such that the absorbent needled felt is useable to be in direct contact with the wound in use, the dressing further comprising a further elastomeric hydrocolloid adhesive layer located on a proximal surface of the dressing for adhering the proximal surface of the dressing to the skin of a user, wherein the further elastomeric hydrocolloid layer is windowed so as to allow the absorbent layer to be directly contactable with the wound in use.

In embodiments, the non-woven resilient sheet has a thickness of from about 0.1 mm to about 0.14mm, such as 0.12mm and / or the average distance between adjacent flutes of the fluted configuration is from about 4mm to about 5mm. The average flute height of the fluted configuration may be from about 0.1mm to about 7.0mm, 0.2mm to about 4mm or from about 0.5mm to about 3.5mm. Preferably, the gelling fibres may include 90-95 wt% of the alginate, 3-5 wt% of the carboxymethylcellulose (CMC) and 2-4 wt% of the silver carbonate relative to the dry mass weight of the gelling fibres. According to further embodiments, the alginate may have a mannuronic acid : guluronic acid ratio of from about 90: 10 to about 50 : 50, about 80: 20 to about 50 : 50 or about 70: 30 to about 50 : 50. In embodiments, the absorbent non-woven material may have a thickness of from about 2.6mm to about 3.0mm. Preferably, the elastomeric backing layer may be a perforated polyurethane backing film, optionally having a thickness of about 10$\mu$m, about 11$\mu$m, about 12$\mu$m, about 13$\mu$m, about 14$\mu$m, about 15$\mu$m, about 16$\mu$m, about 17$\mu$m, about 18$\mu$m, about 19$\mu$m or about 20$\mu$m. Even more preferably, the elastomeric backing layer has a thickness of about 15$\mu$m.

In some embodiments of the invention, the overall thickness of the resilient wound dressing may be from about 1mm to about 10mm, preferably from about 3mm to about 5mm, e.g. about 4mm.

## Description of the figures

The present invention will be described with reference to the following non-limiting examples and figures, which show:

**Figure** 1: A schematic view of the steps of carding and needle bonding a non-woven absorbent material (felt) to the non-woven resilient sheet of elastomeric material according to the present invention.

**Figures 2a and 2b:** A side view of an absorbent layer as provided in preferred embodiments of the first aspect of the invention, or an absorbent article according to the fourth or fifth aspects of the invention and producible according to the method of the second aspect of the invention.

**Figure** 3: A plan view of a wound dressing according to the present invention depicting a windowed elastomeric adhesive layer applied to the proximal surface of the absorbent layer.

**Figure 4:** A figurative cross-sectional view of a resilient wound dressing of the present invention.

**Figure 5:** A stretch force plot (i.e. Force (N) vs Strain (%)) for an exemplary wound dressing of the invention stretched in the longitudinal direction.

**Figure 6:** A stretch force plot (i.e. Force (N) vs Strain (%)) for a comparative Surgical wound dressing not according to the invention stretched in the longitudinal direction.

**Figure 7:** A stretch force plot (i.e. Force (N) vs Strain (%)) for an exemplary wound dressing according to the invention stretched in the lateral (cross) direction.

**Figure 8:** A stretch force plot (i.e. Force (N) vs Strain (%)) for a comparative Surgical wound dressing not according to the invention stretched in the lateral (cross) direction.

## Methods and detailed description

### Preparation of the resilient wound dressing

An exemplary method of manufacturing a resilient wound dressing according to the present invention is provided below.

### Manufacture of the non-woven absorbent material

An aqueous spinning dope was prepared containing a formulation of sodium alginate, CMC and dispersed particles of silver carbonate. The dope was prepared by initially mixing the silver carbonate particles with water until the silver compound is fully dispersed. The CMC and sodium alginate components were then added before undergoing high shear mixing until a uniform dope mixture was obtained. The resulting dope mixture was then allowed to degas to let air bubbles in the mixture escape. The degassed dope mixture was then filtered to remove large particles (i.e. to remove particles over 35 microns). The filtered dope was then pumped through a spinnerette (e.g. hole size 70 microns) into a coagulant bath containing of 2%-4% w/w calcium chloride dehydrate coagulant solution to form coagulated fibres. The resulting fibres (i.e. the tow) were then orientated by stretching the tow in hot water (90°C) before then washing with water to remove residual salts formed via ion exchange during coagulation. A solvent wash was then performed using suitable solvents (acetone/water mixtures). The washed fibres were then dried in a hot air oven and may be optionally finished using a finishing agent (such as Polyethylene Glycol - PEG400). The fibres were then cut into staple lengths, carded and needled using techniques known in the art to form a non-woven absorbent felt material via fibre entanglement.

### Manufacture of the absorbent layer by needle bonding of the a non-woven absorbent material (felt) to the non-woven resilient sheet of elastomeric material

A sheet of melt-blown resilient non-woven polyurethane (M1630 White 500/1550 Stretch Non-Woven supplied by Freudenberg) was laid under tension and needle bonded to the non-woven absorbent felt material described above to form an absorbent layer.

Figure 1 illustrates an exemplary in line method of carding and needle bonding the a non-woven absorbent material (felt) to the non-woven resilient sheet of elastomeric material according to the present invention . As shown in Figure 1 a needling line (1) using two needle looms (3) and (7) (needle bonding stations) is used to manufacture the absorbent layer of the present invention. Firstly, a layered fibrous web (2) produced as a result of the carding (described above) is fed into the first needle loom (3) and the absorbent fibres are needled to form the non-woven absorbent felt (4) material via fibre entanglement also described above.

Subsequently, a sheet of melt-blown resilient non-woven polyurethane (e.g. M1630 White 500/1550 Stretch Non-Woven supplied by Freudenberg) (5) is introduced under tension on top of the non-woven absorbent felt material (4). The applied tension ensures that the material is stretched at least in the longitudinal direction in this embodiment. The tension control is achieved using a tension control unwind and a nip roller set up (6). The tensioned sheet is then needle bonded in the second needle loom (7) to create laminate stretchy absorbent layer (8) with a fluted appearance once relaxed. The absorbent layer is rewound to form a roll. It will be appreciated that the needle felting of the absorbent layer may alternatively be performed separately in a remote location.

Figures 2a and 2b illustrate a stretchy absorbent layer obtainable using the method described above, such as using the production equipment as illustrated in Figure 1. The fluted configuration (8a) of the wound dressing can be seen. The distance between each flute is represented by distance "x" and the flute height is represented by distance "y". In use, when typically forming part of the overall resilient wound dressing as described herein, the fibrous fluted configuration (8a) can stretch (or expand - as depicted by the double-headed arrows in Figures 2a and 2b) together with the resilient non-woven polyurethane (8b) web to which it is needle bonded in response to a stretching force applied. This can occur when the resilient dressing of the present invention is applied to a wound located at a joint of the body (e.g. knee or elbow) wherein motion of the joint can apply a stretching force to the dressing. This force causes the lengthening of the bonded layers (8a) and (8b) as well as flattening of the flutes (8a) during the stretching process. Once the force is removed or subsides, the bonded layers (8a) and (8b) then contract to their pre-stretched or pre-expanded state, leading to the re-gathering of the absorbent material into a fluted configuration again. This stretching mechanism enhances the conformability of the dressing to the anatomy and profile of the wound when located at a joint.

### Manufacture of the elastomeric adhesive layer

A suitable hydrocolloid roll stock (e.g. sodium carboxymethylcellulose, polysaccharides and pectin containing hydrocolloid roll stocks) is mixed at a temperature of 80°C-120°C to form a hydrocolloid dope. This may be done, for example, using a Z-blade mixer. The hydrocolloid dope is then shaped into hydrocolloid logs for downstream processing. The hydrocolloid logs are then extruded through a heated die with a set aperture at 90°C-120°C onto a suitable carrier liner (e.g. paper or film) to form a flexible hydrocolloid sheet matrix. The suitable elastomeric adhesive layer (9) (e.g. hydrocolloid sheet matrix) is then cast onto a suitable elastomeric backing layer (10) (e.g. a polyurethane backing film) ready for adhering to the absorbent layer during the assembly of the dressing. The elastomeric adhesive layer and elastomeric backing layer are depicted bonded together in Figure 4. Preferably, the backing layer has waterproof and bacterial barrier properties.

*Manufacture of the resilient wound dressing assembly*

The elastomeric backing layer (10) with the elastomeric adhesive layer (9) in the form of cast hydrocolloid sheet matrix cast thereon (as described above) is laid onto a conversion line. A fluted absorbent layer (8) as described above is cut to shape and placed onto the elastomeric backing layer (10) with the cast hydrocolloid sheet matrix (9) cast thereon to adhere the backing layer (10) to the absorbent layer (8) via the hydrocolloid sheet to form the resilient wound dressing assembly. Preferably the resilient sheet of elastomeric material is orientated to face the adhesive surface such that the absorbent needled felt is facing away from the adhesive in a proximal (ultimately wound facing) direction.

A further elastomeric adhesive layer in the form of a second hydrocolloid sheet matrix (produced as described above) may then be used to form a windowed or apertured hydrocolloid sheet matrix. If provided, such an elastomeric adhesive layer (11) is then laid over the resilient wound dressing assembly described above to form a laminated structure (depicted by layers 8, 9, 10 and 11 in Figure 4). Optional, handle or release liners (12) are also included as shown in Figure 4.

A plan view of the laminate structure is illustrated in Figure 3. Here, the fluted configuration (8a) can be seen through the window or central aperture (13) formed in the elastomeric adhesive layer (11). During use, the fluted configuration (8a) is configured to be directly exposed to the wound whilst the elastomeric adhesive layer (11) is able to adhere the dressing to the area around the wound. This way, the fluid (e.g. wound exudate) can directly flow onto the flutes before being retained by the overall absorbent layer (8).

Figure 4 illustrates a figurative cross-sectional view of an exemplary wound dressing of the present invention. As shown, the wound dressing includes a laminate structure having an elastomeric backing layer (10) adhered, via an elastomeric adhesive layer (9), to an absorbent layer (8), which comprises a non-woven absorbent layer (8a) bonded to a non-woven resilient sheet of elastomeric material (8b) wherein the resilient sheet is positioned distal portion of the absorbent layer (8) thus allowing the non-woven absorbent material (8a) to be proximal to the wound, and preferably configured to be in direct contact with the wound. The absorbent layer includes, on its distal (wound facing) surface, a further elastomeric adhesive layer (11) in the form of windowed hydrocolloid sheet matrix which is protected prior to use with a release or handle liner (12). During use the operator removes the handle or release liner (12) before carefully placing the wound dressing onto the wound site to be dressed. The wound dressing adheres and attaches to the skin surrounding the wound via the windowed second hydrocolloid sheet matrix (11). In the depicted embodiment, the central aperture (13) created by the windowed hydrocolloid sheet matrix exposes a fluted absorbent layer (8) to the wound site. This allows direct contact between fluted absorbent layer (8) and the wound exudate whilst at the same time ensuring that the dressing is firmly securely to the wound site.

## Experimental Tests

A series of test were performed to measure the performance of an exemplary resilient wound dressing of the present invention against a comparative commercial wound dressing not according to the invention (i.e. Aquacel Ag Surgical wound dressing marketed by ConvaTec). The Aquacel Ag Surgical wound dressing includes a backing layer, absorbent layer and two hydrocolloid layers. One hydrocolloid layer adheres the absorbent layer to the backing layer and the other adheres the absorbent layer to the wound site during use. The absorbent layer contains a non-woven material made 100% CMC with silver. Elastic yarns are stitched through the absorbent layer in the longitudinal direction (warp) and non-elastic yarns are stitched in the cross direction (weft).

All test methods and procedures used to obtain the test data are provided herein and, unless specified otherwise, are known to the person skilled in the art.

The exemplary resilient wound dressing of the present invention used for the purposes of the following tests is referred to herein as the AMS wound dressing. The AMS wound dressing may be manufactured using the exemplary method of manufacturing a resilient wound dressing provided above and the material described below. The dope mixture used to provide the non-woven absorbent material is described below in Table 1 with the sheet of melt-blown resilient non-woven polyurethane being M1630 White 500/1550 Stretch Non-Woven supplied by Freudenberg.

Table 1

| Component | % By Weight |
|---|---|
| Sodium Alginate (M:G ratio 60:40) | 92 |
| CMC (Carboxymethyl Cellulose) | 4.25 |
| Silver Carbonate 99.9% | 3.75 |

The hydrocolloid used in the AMS wound dressing may be manufactured and formulated using techniques and procedures known in the art. An exemplary hydrocolloid for use in the embodiments of the invention is described in Table 2 below. This hydrocolloid composition was the elastomeric adhesive layer used in the tested AMS wound dressing described herein.

Table 2

| Hydrocolloid | Component Amount (wt%) |
|---|---|
| Polyisobutylene | 28.36 |
| Carboxymethylcellulose | 29.63 |
| Poly(styrene-butadiene-styrene) Block Copolymer | 16.29 |
| Hydrocarbon Resin | 10.40 |
| Purified Powdered Cellulose, Flock | 8.44 |
| Pectin | 6.35 |
| Other ingredients (including preservative and liquid phase oil) | 0.53 |

The elastomeric backing layer used in the AMS wound dressing described herein was 15$\mu$m Polyurethane Pink film (Inspire 2150) supplied by Coveris.

A series of tests were performed on the AMS wound dressing and the resulting Absorbency Free Swell, Retention under Load, MVTR, Total Fluid Handling, Extensibility, Permanent Set (%), Peel Adhesion (N/2.5cm), Coefficient of Friction, Waterproofness and Conformability - Bending (mm) data can be found presented in Table 3 below.

Table 3

| Test | AMS wound dressing | Performance of AMS wound dressing |
|---|---|---|
| Absorbency Free Swell (g/100cm$^2$) | 33.8 | Good absorbency performance |
| Retention under Load (g/100cm$^2$) | 25.8 | Good retention performance |
| MVTR (g/m$^2$/24hrs) | 2288 | Excellent breathability performance |
| Total Fluid Handling (g/10cm$^2$/24hrs) | 8.3 | AMS dressing demonstrates excellent fluid handling |
| Extensibility (N/cm) | 3.0 | Good extensibility force demonstrated |
| Permanent Set (%) | 1% | Good elasticity |
| Peel Adhesion (N/2.5cm) | 15.4 | AMS dressing demonstrates good peel adhesion |
| Thickness (mm) | 2.48 | AMS dressing has a slim and lightweight design |
| Coefficient of Friction (N) | 1.09 | AMS dressing demonstrates a low friction profile (i.e. reduced rucking and pulling under clothing or bedsheets) |
| Waterproofness | YES | AMS dressing is waterproof and prevents ingress of water or leakage of wound fluids |
| Conformability - Bending (mm) | 19.1 | AMS dressing demonstrates low bending length translating into excellent conformability around anatomical contours. |

Table 4 presents certain test data for the AMS dressing for comparison against the Aquacel Ag Surgical dressing.

Table 4

| Test | Aquacel Ag Surgical | AMS wound dressing | Comparison comments |
|---|---|---|---|
| MVTR (g/m²/24hrs) | 531 | 2288 | AMS dressing demonstrates significantly improved breathability as compared to Aquacel. |
| Total Fluid Handling (g/10cm²/24hrs) | 6.3 | 8.3 | AMS dressing demonstrates improved fluid handling |
| Peel Adhesion (N/2.5cm) | 10.50 | 15.4 | AMS dressing demonstrates improved adhesion (i.e. the dressing remains in place for intended wear time) as compared to Aquacel. |
| Thickness (mm) | 3.18 | 2.48 | AMS dressing has a slimmer design and profile as compared to Aquacel. |
| Longitudinal stretch test | Failure before 100% stretch | No failure even at 100% stretch | AMS dressing shows significantly improved stretch properties |
| Lateral stretch test | Failure before 50% stretch | No failure even at 100% stretch | AMS dressing shows significantly improved stretch properties |

As illustrated by the data in Table 4, the AMS wound dressing embodiment according to the present invention possesses at least superior MVTR, Total Fluid Handling, Peel Adhesion, thickness and stretch parameters as compared to the Aquacel Ag Surgical dressing.

***Absorbency Free Swell***

The absorbency free swell (i.e. the absorbency under no-load) data in Table 3 was obtained using a method which is aligned with BS EN 13726-1:2002 *Test method for primary wound dressings, part 1: aspects of absorbency, section 3.2, for use in R&D and QC laboratories.* The procedure required the AMS wound dressing to be cut into 5cm by 5cm squares using a cutting die. A Petri dish was placed on a balance and the balance is tared. The sample of AMS dressing was then placed in the Petri dish and its mass recorded ($W_1$). Then, 40 times the mass of the dressing of Solution A (a standard test solution used in wound care containing 142 millimoles of sodium ions and 2.5 millimoles of calcium ions dissolved in distilled water (made as per ISO 13726-1:2002)), pre-warmed to a temperature of $37 \pm 1$ °C, was added to the Petri dish.

The sample, in its Petri dish, was then placed into an environmental chamber at $37 \pm 1$°C for $30 \pm 1$ minutes, recording the temperature at the start and end of the incubation. The samples were removed from the chamber and suspended by one corner for 30 seconds using forceps. The sample was weighed and the mass recorded ($W_2$).

The absorbency of the AMS wound dressing sample was calculated according to the following formula:

$$\text{Absorbency} = (W_2 - W_1) \times 4 \text{ (Units: g/100 cm}^2)$$

***Retention under load***

The retention under load data in Table 3 is a measure of how effectively the wound dressing retains wound exudate under pressure as it may be in use (e.g. under compression bandaging). The weight applied to the samples is between 1366.4g and 1374.0g and corresponds to a minimum pressure of 40 mmHg (equal to that applied by a compression bandage at the ankle in the case of venous leg ulcers). The procedure begins as described under Absorbency Free Swell, above. After $W_2$ is obtained, the sample is placed in a tray with a mesh base, which is in turn placed in a larger collection tray. A 5cm by 5cm Perspex plate is placed squarely on the sample, onto which a stainless steel weight is placed for a period of $30 \pm 2$ seconds. After removing the weight and Perspex plate, the sample is weighed to obtain $W_3$.

The retention under load of the AMS wound dressing sample was calculated according to the following: ***Total Fluid Handling***

$$\text{Retention} = (W_3 - W_1) \times 4 \text{ (Units: g/100 cm}^2)$$

*Total Fluid Handling*

The Total Fluid Handling (TFH), Moisture Vapour Transmission Rate (MTVR) and Fluid Absorbance data presented in Table 3 was obtained using a method which is aligned with ISO 13726-1:2002 *Test Methods for Primary Wound Dressings - Part 1: Aspects of Absorbent section 3.3 Fluid Handling Capacity.* The procedure requires a circle of the AMS wound dressing to be cut to have a 55cm diameter. The circle of AMS wound dressing was then attached to the flange of a Paddington Cup (a hollow cup with a 10 cm2 cross section) and secured in place with a retaining ring (e.g. screw top). The weight of the AMS wound dressing and the Paddington Cup is measured (W1) and recorded using a calibrated balance. Then 20ml of Solution A is added to the Paddington Cup and the screw top applied. Solution A is a standard test solution used in wound care contains 142 millimoles of sodium ions and 2.5 millimoles of calcium ions dissolved in distilled water (made as per ISO 13726-1:2002). The Paddington Cup is positioned such that the adhesive side of the AMS wound dressing is in contact with Solution A. The Paddington Cup is then weighed again (W2) and the weight is recorded. The Paddington Cup is placed into a 37°C environmental chamber for 24 hours. Upon removal, the Paddington Cup is left to stand for 30 minutes and the weight recorded (W3). The solution is drained and the Paddington Cup is inverted for 15 minutes before having the weight of the cup measured and recorded for the final time (W4).

The Moisture Vapour Transmission Rate (MVTR) and Fluid Absorbance for the AMS wound dressing sample was calculated according to the following.

$$MVTR = W2 - W3 \times 1000 \text{ (Units: g/m2/24hrs)}$$

$$Fluid\ Absorbance = W4 - W1 \text{ (Units: g/10cm2/24hrs)}$$

The total fluid handling is the sum of MVTR and Fluid absorbance values.

**Peel Adhesion**

The peel adhesion data was obtained using a method which is aligned with ASTM D6282-11.

A sample of the AMS wound dressing was cut to 25mm x 100mm using a cutting press and die. The sample was tape wrapped around one end to form a tab for use with the tensometer. A stainless steel plate was then cleaned with isopropyl alcohol wipes or acetone. The release liner of the cut sample is removed and the sample is applied to the stainless steel plate. The sample and plate were placed on a calibrated 'roll down' machine at a speed of 12inch/min. The stainless steel plate was mounted onto the test rig of a tensometer (Zwick model) so that the tab created is secured in the upper jaw at a 90 degree angle. The upper jaw was moved up, peeling the adhesive from the stainless steel plate at a rate of 254mm/min. The maximum force and the average force are measured by the tensometer in Newtons.

**Thickness tests**

The total thickness of the wound dressing is measured using a calibrated digital calliper and the measurement is recorded at three points on the dressing. The measurement is taken with release liners on wound facing surface of the dressing in order to avoid the equipment becoming tacky. This also reduces the risk of the calliper compressing the adhesive layer and thus giving a false lower reading. The release liners are then removed and measured using the same calliper at three points on the release liner. The final thickness of the wound dressing is calculated as the average thickness of the dressing (as measured with the release liners) minus the average thickness of the release liners.

**Conformability tests**

The conformability data presented in Tables 3 above was obtained using the following method.

The method used to obtain the conformability presented herein is aligned with ISO EN 13426-4:2003 *Test methods for primary wound dressings - Part 4: Conformability.* This method sees a sample of the wound dressing cut in to a rectangular strip. For the AMS dressing the strip was cut from the fluted section of the wound dressing having a width of 2.5cm. The strip then marked (i.e. has two pieces of tape wrapped around either of its ends) approximately 100mm apart. The release liners of the dressing are then removed, if necessary, and the product is left to relax for a minimum of 300 seconds. The distance between the two pieces of tape is measured using a digital calliper and recorded as L1. The sample is then mounted into a tensometer (a Zwick Roell machine was used) and clamped into the jaws of the machine via the taped ends (the jaws are set to be 10mm wider than the marks). The sample is extended by 20% at a rate of 300mm/min and is held at the maximum extension for 60 seconds. The force to extend by 20% is recorded by the tensometer as the Fmax/Maximum force (Newtons). After 60 seconds the sample is released from the jaws of the tensometer and left to relax for a further 300 seconds. The distance between the taped ends is measured again using the digital callipers and recorded as L2.

*Extensibility*

Extensibility is defined as the force required to stretch a wound dressing sample to a known extension. To calculate the extensibility the Fmax is divided by 2.5cm.

*Permanent Set*

Permanent set is defined as the increase in length of a sample after the stretching and relaxing expressed as a percentage of the original length. The permanent set is calculated as follows:

$$\text{Permanent Set } (\%) = ((L2 - L1) / L1) \times 100$$

Conformability is an important factor for wound dressings that are in contact with the skin in the region of moveable joints, such as the elbow and knee, as the higher the conformability the better the ability of the wound dressing to adapt to the shape and movement of the body. The measurement of the permanent set provides an assessment of the memory of a wound dressing and ability of a wound dressing to flex and contract. The force required to stretch the wound dressing is also represented by the Fmax.

*Conformability - Bending length*

Bending length is defined as the length of sample material of predetermined width that is pushed through the cantilever conformability tester (Shirley stiffness tester) when the tester reaches a bending angle of 41.5°. The bending length data in Table 3 was obtained using a method aligned with *ASTM D1388 Standard Test Method for Stiffness of Fabrics option A - Cantilever Test.* A sample of the AMS dressing was cut to 25 mm wide and 140 mm long using a die cutter. The mass of the sample was determined using a calibrated balance and recorded (M).
The sample is aligned with the line scribed on the edge of the platform of the Shirley stiffness tester and the removable slide placed on the sample. The clamped sample is then moved by hand at a rate of approximately 120mm/min +5% until the edge of the sample touches the knife edge, which is set at an incline of 41.5°. The overhang length of the sample from the linear scale is measured to the nearest 0.1 cm and recorded.
The bending length of the AMS wound dressing sample was calculated according to the following equation:

$$\text{Bending length} = \text{length of overhang}/2 \text{ (Units: mm)}$$

*Stretch force tests*

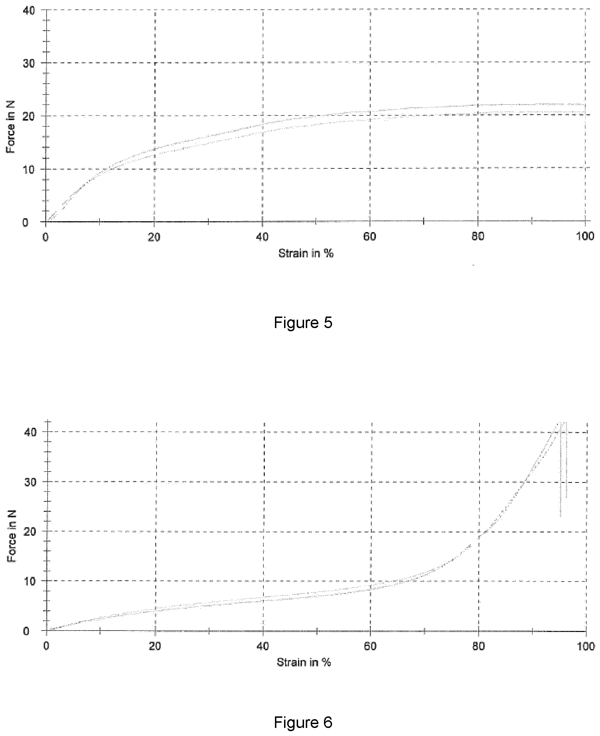

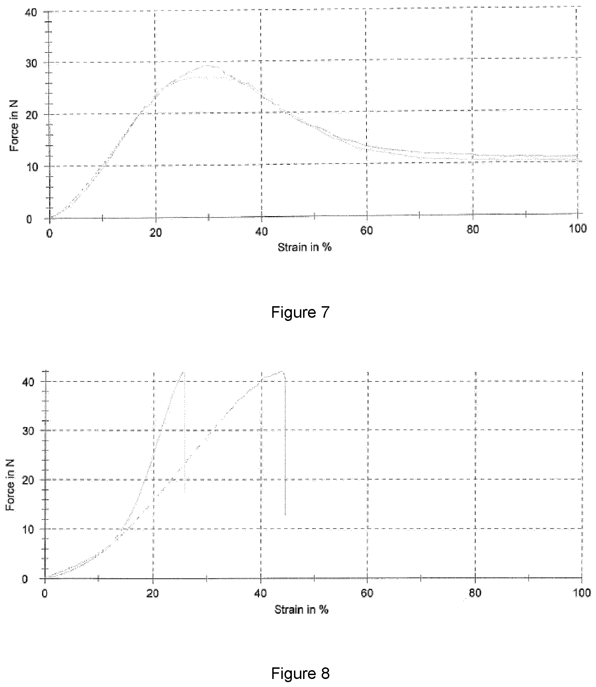

Stretch force tests were performed on the AMS wound dressing and the Aquacel Ag Surgical samples both in the longitudinal and cross direction. This was done to investigate the multidirectional stretching ability of the respective wound dressings.
Test rectangles 25mm x 60mm were cut from the samples ensuring that the central absorbent areas of the dressings were used. Any release liners were removed. The samples were then mounted onto a tensometer (a Zwick Roell machine was used) before then being stretched in the cross direction and the longitudinal direction. The strain profiles for each of the samples when stretched in the cross (lateral) direction and the longitudinal directions are measured as Force vs % Strain (see Figures 5-8). Each samples was tested twice.
As illustrated in Figure 5, when stretched in the longitudinal direction, the AMS wound dressing exhibits a gradual increase in the force require to reach a 50% strain, which then plateaus and maintains substantially constant until a 100% strain is achieved. The maximum force achieved during the longitudinal stretching of the AMS wound dressing averaged at 21.3N. In contrast, as seen in Figure 6, the Aquacel Ag Surgical sample in the longitudinal direction required lower forces during initial stretching to cause an increase in the strain. However, the Aquacel Ag Surgical ultimately required dramatic increase in the force above 60% strain before then reaching material failure. It is believed that this shows that the Aquacel Ag Surgical reached the elastic limit of its stitch-bonded configuration leading to the material failure.
As illustrated in Figure 7, when stretched in the cross direction, the AMS wound dressing required a moderate force to effect strain in the initial period but without any material failure. The maximum force achieved during the cross direction stretching of the AMS wound dressing averaged at 28.2N. The gradual increase and decrease of the force required to achieve 100% strain suggests that an initial structural separation of fibres within the dressing occurred but without material failure. The Aquacel Ag Surgical sample in the cross (lateral) direction, as shown in Figure 8, requires a rapid increase in force until the point of material failure which occurs before 50% strain has been achieved.

The data illustrated in Figures 5-8 illustrate the superior multidirectional stretching characteristics of the AMS wound dressing. Figures 5 and 7 show that the wound dressings of the present invention are able to reach 100% both in the longitudinal and lateral (cross) directions, requiring a moderate amount of force and without experiencing any material failure.

### *Coefficient of Friction*

The coefficient of friction of the exterior surface (i.e. outward or non-wound facing surface) of the backing layer within the AMS wound dressing was measured using a Zwick Roell Tensometer equipped with a 50N load cell and utilising the testXpert II software and method: *"WI-405 Coefficient of Friction".* A sample of the AMS wound dressing was cut using a 63 by 100mm rectangular die. The sample was wrapped around the metal plate with the surface to be analysed facing outwards, and clamped into place. The load cell was then connected to the metal plate via the thread. The force was then zeroed and the test begun. On completion of the measurement, the metal plate was removed and the sample disposed of.

### *Waterproofness*

The waterproofness data presented in Table 3 above was obtained using the following method. The method used to obtain the waterproofness presented herein is aligned with standard method BS EN 13726-3:2003 *Non-active medical devices - test methods for primary wound dressings - Part 3: Waterproofness.* The procedure determines if the dressing is waterproof, waterproof being defined as being able to withstand a hydrostatic head of 500mm of water for 5 minutes. The procedure requires a circle of the AMS wound dressing to be cut to have a diameter of 90 mm.

A rubber ring is placed on the circular opening of the cell, which is connected to a burette using appropriate tubing. The cell is filled with distilled/deionized water at $21 \pm 2°C$ (dispensed at this temperature) to the top of the rubber ring using the burette. Any water present on the cell or rubber ring is then dried and any air bubbles in apparatus removed to ensure there are no impediments to the flow of water. The dressing was slid onto the rubber ring with the external side in contact with the rubber ring. A clean, dry filter paper was attached to the wound contact side of the dressing and a second rubber ring placed over the top. A metal ring was placed over the second rubber ring and secured with four G-cramps.

The burette was filled with water and the system left for 5 minutes. After this time the filter paper was examined for water penetration. If water was present on the filter paper, the dressing had failed the test as water had penetrated it. If water was not present on the filter paper, the dressing has passed the test as water had not penetrated it.

### Claims

1.    A resilient wound dressing comprising:

> an absorbent layer comprising a non-woven resilient sheet of elastomeric material bonded to a non-woven absorbent material;
> an elastomeric backing layer; and
> an elastomeric adhesive layer located between the absorbent layer and the backing layer to adhere the absorbent layer to the backing layer,
> wherein the non-woven resilient sheet and the non-woven absorbent material are bonded by needle bonding.

2.    The resilient wound dressing according to claim 1, wherein:

> a) the non-woven resilient sheet comprises a melt-blown polymer web, optionally wherein the melt-blown polymer web comprises polyurethane fibres; and/or
> b) the absorbent non-woven material is an absorbent non-woven fabric, preferably a needled felt, optionally wherein the fabric comprises gelling fibres, and optionally further wherein:
>
> > i) the gelling fibres comprise an alginate, carboxymethylcellulose (CMC), carboxymethyl viscose, gelatine, pectin, hydroxyethyl cellulose, hydroxypropyl cellulose, methyl cellulose, sulphonated cellulose (SC), sulphonated viscose, carboxymethyl chitosan, polyvinyl alcohol or any combination thereof; and/or
ii) the gelling fibres comprise an alginate and carboxymethylcellulose (CMC), preferably wherein the alginate and carboxymethylcellulose (CMC) are co-spun to form fibres which each comprise the alginate and carboxymethylcellulose (CMC).

3. The resilient wound dressing according to any one of the preceding claims wherein the non-woven absorbent material further comprises an anti-microbial agent and/or anti-biofilm agent; optionally wherein the anti-microbial agent and/or anti-biofilm agent is selected from the group consisting of silver, silver compounds, iodine compounds, monoguanides, biguanides, cationic surfactants, biocides, dispersing agents and a combination thereof, and optionally further wherein the anti-microbial agent and/or anti-biofilm agent is a silver compound, preferably silver carbonate.

4. The resilient wound dressing according to any one of the preceding claims wherein:

the elastomeric backing layer is a polyurethane backing film, preferably a perforated polyurethane backing film; and/or
the resilient wound dressing further comprises a further elastomeric adhesive layer located on a proximal surface of the dressing for adhering the proximal surface of the dressing to the skin of a user, preferably wherein the further elastomeric adhesive layer is windowed; and/or
the elastomeric adhesive layer and/or further elastomeric adhesive layer independently comprises a hydrocolloid, optionally wherein the hydrocolloid comprises sodium carboxymethylcellulose, polysaccharides and pectin; and/or
the non-woven absorbent material is in a fluted configuration.

5. A method of manufacturing a resilient wound dressing according to any previous claim comprising:

(a) bonding the non-woven resilient sheet to the non-woven absorbent material to form the absorbent layer, wherein the bonding comprises needle bonding; and
(b) adhering the absorbent layer to the elastomeric backing layer using the elastomeric adhesive layer.

6. A method of manufacturing a resilient absorbent article for a wound dressing comprising:

expanding a non-woven resilient sheet of elastomeric material from a pre-expanded state to an expanded state; and
bonding a non-woven absorbent material to the expanded non-woven resilient sheet, wherein the bonding comprises needle bonding; and
allowing the non-woven resilient sheet to return from the expanded state towards the pre-expanded state to provide the resilient absorbent article.

7. The method according to claim 5, wherein bonding step (a) comprises:

expanding the non-woven resilient sheet of elastomeric material from a pre-expanded state to an expanded state; and
bonding the non-woven absorbent material to the expanded non-woven resilient sheet, wherein the bonding comprises needle bonding; and
allowing the non-woven resilient sheet to return from the expanded state towards the pre-expanded state to provide the absorbent layer.

8. The method according to claim 6, wherein the method further comprises adhering the absorbent article to an elastomeric backing layer using an elastomeric adhesive layer.

9. The method according to any one of claims 5 to 8,

wherein following the bonding of the non-woven resilient sheet to the non-woven absorbent material, the non-woven absorbent material adopts a fluted configuration; and / or
wherein the non-woven resilient sheet comprises a melt-blown polymer web, optionally wherein the melt-blown polymer web comprises polyurethane fibres.

**10.** The method according to any one of claims 5 to 9, wherein the absorbent non-woven material is a non-woven absorbent fabric, preferably wherein the fabric comprises gelling fibres, and optionally further wherein the gelling fibres comprise an alginate, carboxymethylcellulose (CMC), carboxymethyl viscose, gelatine, pectin, hydroxyethyl cellulose, hydroxypropyl cellulose, methyl cellulose, sulphonated cellulose (SC), sulphonated viscose, carboxymethyl chitosan, polyvinyl alcohol or any combination thereof; optionally wherein the gelling fibres comprise an alginate and carboxymethylcellulose (CMC), preferably wherein the alginate and carboxymethylcellulose (CMC) are co-spun to form fibres which each comprise the alginate and carboxymethylcellulose (CMC).

**11.** The method according to any one of claims 5 to 11, wherein the non-woven absorbent material further comprises an anti-microbial agent and/or anti-biofilm agent; optionally wherein the anti-microbial agent and/or anti-biofilm agent is selected from the group consisting of silver, silver compounds, iodine compounds, monoguanides, biguanides, cationic surfactants, biocides, dispersing agents and a combination thereof.

**12.** The method according to claim 11, wherein the anti-microbial agent and/or anti-biofilm agent is a silver compound, preferably silver carbonate; optionally wherein the gelling fibres comprise an alginate, carboxymethylcellulose (CMC) and a silver compound, preferably wherein the alginate, carboxymethylcellulose (CMC) and silver compound are co-spun together to form fibres which each comprise the alginate, carboxymethylcellulose (CMC) and the silver compound.

**13.** The method according to any one of claims 5 to 12, further wherein the elastomeric backing layer is a polyurethane backing film, preferably a perforated polyurethane film; and/or the method further comprises applying a windowed elastomeric adhesive layer to a proximal surface of the absorbent layer or article.

**14.** A resilient absorbent article comprising an absorbent non-woven material needle bonded to a resilient sheet of elastomeric material such that the absorbent article adopts a fluted configuration when the absorbent article is not under tension and is able to adopt a substantially flattened configuration when the absorbent article is under tension; optionally wherein the resilient sheet of elastomeric material is a non-woven resilient sheet of elastomeric material.

**15.** The wound dressing according to claim 1 comprising an absorbent layer, an elastomeric polyurethane backing film; and an elastomeric hydrocolloid adhesive layer located between the absorbent layer and the backing layer to adhere the absorbent layer to the backing layer, the absorbent layer comprising a melt-bonded sheet of elastomeric material needle bonded to an absorbent needled felt comprising gelling fibres, preferably wherein the fibres comprise alginate, carboxymethylcellulose (CMC) and particles of silver carbonate co-spun together to form fibres which each comprise the alginate, carboxymethylcellulose (CMC) and silver carbonate particles, optionally wherein

the elastomeric backing layer is porous (e.g. perforated and the absorbent layer is orientated in the dressing such that the resilient melt-bonded elastomeric sheet is positioned distal to the wound in use relative to the absorbent needled felt and the absorbent needled felt is configured so as to be able to be in direct contact with the wound in use, and optionally wherein

the wound dressing comprises an absorbent layer, an elastomeric porous polyurethane backing film; and an elastomeric hydrocolloid adhesive layer located between the absorbent layer and the backing film to adhere the absorbent layer to the backing film, the absorbent layer consisting of a melt-bonded web of an elastomeric polyurethane which is needle bonded to an absorbent needled felt comprising gelling fibres, wherein the fibres comprise alginate, carboxymethylcellulose (CMC) and particles of silver carbonate co-spun together to form fibres which each comprise the alginate, carboxymethylcellulose (CMC) and silver carbonate particles), wherein the absorbent layer is orientated in the dressing such that the resilient melt-bonded elastomeric web is positioned distal to the wound when in use relative to the absorbent needle felt which is positioned relatively more proximal to the wound when in use and the dressing is configured such that the absorbent needled felt is useable to be in direct contact with the wound in use, the dressing further comprising a further elastomeric hydrocolloid adhesive layer located on a proximal surface of the dressing for adhering the proximal surface of the dressing to the skin of a user, wherein the further elastomeric hydrocolloid layer is windowed so as to allow the absorbent layer to be directly contactable with the wound in use.

**Patentansprüche**

**1.** Elastischer Wundverband, Folgendes umfassend:

eine absorbierende Schicht, umfassend ein Blatt aus elastischem Vlies aus Elastomermaterial, welches an ein absorbierendes Vliesmaterial gebunden ist;

eine Elastomer-Verstärkungsschicht; und

eine Elastomer-Klebeschicht, welche zwischen der absorbierenden Schicht und der Verstärkungsschicht angeordnet ist, um die absorbierende Schicht an der Verstärkungsschicht anzukleben,

wobei das elastische Vliesblatt und das absorbierende Vliesmaterial durch Vernadeln gebunden sind.

2. Elastischer Wundverband nach Anspruch 1, wobei:

a) das elastische Vliesblatt eine schmelzgeblasene Polymerbahn umfasst, wobei optionsweise die schmelzgeblasene Polymerbahn Polyurethanfasern umfasst; und/oder

b) das absorbierende Vliesmaterial ein absorbierender Vliesstoff ist, vorzugsweise ein genadelter Filz, wobei optionsweise der Stoff gelierende Fasern umfasst, und wobei optionsweise ferner:

i) die gelierenden Fasern ein Alginat, Carboxymethylcellulose (CMC), Carboxymethylviscose, Gelatine, Pektin, Hydroxyethylcellulose, Hydroxypropylcellulose, Methylcellulose, sulfonierte Cellulose (SC), sulfonierte Viskose, Carboxymethylchitosan, Polyvinylalkohol oder eine beliebige Kombination davon umfassen; und/oder

ii) die gelierenden Fasern ein Alginat und Carboxymethylcellulose (CMC) umfassen, wobei vorzugsweise das Alginat und die Carboxymethylcellulose (CMC) gemeinsam gesponnen werden, um Fasern zu bilden, welche jeweils das Alginat und die Carboxymethylcellulose (CMC) umfassen.

3. Elastischer Wundverband nach einem der vorhergehenden Ansprüche, wobei das absorbierende Vliesmaterial ferner einen antimikrobiellen Wirkstoff und/oder einen Anti-Biofilm-Wirkstoff umfasst; wobei optionsweise der antimikrobielle Wirkstoff und/oder der Anti-Biofilm-Wirkstoff aus der Gruppe ausgewählt ist, bestehend aus Silber, Silberverbindungen, Iodverbindungen, Monoguaniden, Biguaniden, kationischen oberflächenaktiven Stoffen, Bioziden, Dispersionswirkstoffen und einer Kombination davon, und wobei optionsweise ferner der antimikrobielle Wirkstoff und/oder der Anti-Biofilm-Wirkstoff eine Silberverbindung ist, vorzugsweise Silbercarbonat.

4. Elastischer Wundverband nach einem der vorhergehenden Ansprüche, wobei:

die Elastomer-Verstärkungsschicht eine Polyurethan-Verstärkungsschicht ist, vorzugsweise ein perforierter Polyurethan-Verstärkungsfilm; und/oder

der elastische Wundverband ferner eine weitere Elastomer-Klebeschicht umfasst, welche an einer proximalen Fläche des Verbandes zum Ankleben der proximalen Fläche des Verbandes an der Haut eines Benutzers angeordnet ist, wobei vorzugsweise die weitere Elastomer-Klebeschicht mit Fenstern versehen ist; und/oder

die Elastomer-Klebeschicht und/oder die weitere Elastomer-Klebeschicht unabhängig ein Hydrokolloid umfasst, wobei optionsweise das Hydrokolloid Natriumcarboxymethylcellulose, Polysaccharide und Pektin umfass; und/oder

das absorbierende Vliesmaterial eine gerippte Konfiguration aufweist.

5. Verfahren zum Herstellen eines elastischen Wundverbandes nach einem der vorhergehenden Ansprüche, Folgendes umfassend:

(a) Binden des elastischen Vliesblattes an das absorbierende Vliesmaterial zum Bilden der absorbierenden Schicht, wobei das Binden Vernadeln umfasst; und

(b) Ankleben der absorbierenden Schicht an die Elastomer-Verstärkungsschicht unter Verwendung der Elastomer-Klebeschicht.

6. Verfahren zum Herstellen eines elastischen, absorbierenden Artikels für einen Wundverband, Folgendes umfassend:

Expandieren eines elastischen Vliesblattes aus Elastomermaterial von einem vor-expandierten Zustand in einen expandierten Zustand; und

Binden eines absorbierenden Vliesmaterials an das expandierte elastische Vliesblatt, wobei das Binden Vernadeln umfasst; und

Versetzen des elastischen Vliesblattes in die Lage, vom expandierten Zustand in den vor-expandierten Zustand zurückzukehren, um den elastischen absorbierenden Artikel bereitzustellen.

7. Verfahren nach Anspruch 5, wobei der Schritt des Bindens (a) Folgendes umfasst:

Expandieren des elastischen Vliesblattes aus Elastomermaterial von einem vor-expandierten Zustand in einen expandierten Zustand; und

Binden des absorbierenden Vliesmaterials an das expandierte elastische Vliesblatt, wobei das Binden Vernadeln umfasst; und

Versetzen des elastischen Vliesblattes in die Lage, vom expandierten Zustand in den vor-expandierten Zustand zurückzukehren, um die absorbierende Schicht bereitzustellen.

8. Verfahren nach Anspruch 6, wobei das Verfahren ferner das Ankleben des absorbierenden Artikels an einer Elastomer-Verstärkungsschicht unter Verwendung einer Elastomer-Klebeschicht umfasst.

9. Verfahren nach einem der Ansprüche 5 bis 8,

wobei nach dem Binden des elastischen Vliesblattes an das absorbierende Vliesmaterial das absorbierende Vliesmaterial eine gerippte Konfiguration annimmt; und/oder

wobei das elastische Vliesblatt eine schmelzgeblasene Polymerbahn umfasst, wobei optionsweise die schmelzgeblasene Polymerbahn Polyurethanfasern umfasst.

10. Verfahren nach einem der Ansprüche 5 bis 9, wobei das absorbierende Vliesmaterial ein absorbierender Vliesstoff ist, wobei vorzugsweise der Stoff gelierende Fasern umfasst, und wobei optionsweise die gelierenden Fasern ein Alginat, Carboxymethylcellulose (CMC), Carboxymethylviscose, Gelatine, Pektin, Hydroxyethylcellulose, Hydroxypropylcellulose, Methylcellulose, sulfonierte Cellulose (SC), sulfonierte Viskose, Carboxymethylchitosan, Polyvinylalkohol oder eine beliebige Kombination davon umfassen; wobei optionsweise die gelierenden Fasern ein Alginat und Carboxymethylcellulose (CMC) umfassen, wobei vorzugsweise das Alginat und die Carboxymethylcellulose (CMC) gemeinsam gesponnen werden, um Fasern zu bilden, welche jeweils das Alginat und die Carboxymethylcellulose (CMC) umfassen.

11. Verfahren nach einem der Ansprüche 5 bis 11, wobei das absorbierende Vliesmaterial ferner einen antimikrobiellen Wirkstoff und/oder einen Anti-Biofilm-Wirkstoff umfasst; wobei optionsweise der antimikrobielle Wirkstoff und/oder der Anti-Biofilm-Wirkstoff aus der Gruppe ausgewählt ist, bestehend aus Silber, Silberverbindungen, Iodverbindungen, Monoguaniden, Biguaniden, kationischen oberflächenaktiven Stoffen, Bioziden, Dispersionswirkstoffen und einer Kombination davon.

12. Verfahren nach Anspruch 11, wobei der antimikrobielle Wirkstoff und/oder der Anti-Biofilm-Wirkstoff eine Silberverbindung ist, vorzugsweise Silbercarbonat; wobei optionsweise die gelierenden Fasern ein Alginat, Carboxymethylcellulose (CMC) und eine Silberverbindung umfassen, wobei vorzugsweise das Alginat, die Carboxymethylcellulose (CMC) und die Silberverbindung gemeinsam gesponnen werden, um Fasern zu bilden, welche jeweils das Alginat, die Carboxymethylcellulose (CMC) und die Silberverbindung umfassen.

13. Verfahren nach einem der Ansprüche 5 bis 12, wobei ferner die Elastomer-Verstärkungsschicht ein Polyurethan-Verstärkungsfilm ist, vorzugsweise ein perforierter Polyurethanfilm; und/oder das Verfahren ferner Aufbringen einer mit Fenstern versehenen Klebeschicht auf eine proximale Fläche der absorbierenden Schicht oder des absorbierenden Artikels umfasst.

14. Elastischer, absorbierender Artikel, umfassend ein absorbierendes Vliesmaterial, welches an ein elastisches Blatt aus Elastomermaterial dergestalt gebunden ist, dass der absorbierende Artikel eine gerippte Konfiguration annimmt, wenn der absorbierende Artikel nicht unter Spannung steht und in der Lage ist, eine im wesentlichen abgeflachte Konfiguration anzunehmen, wenn der absorbierende Artikel unter Spannung steht; wobei optionsweise das elastische Blatt aus Elastomermaterial ein elastisches Vliesblatt aus Elastomermaterial ist.

15. Wundverband nach Anspruch 1, umfassend eine absorbierende Schicht, einen Elastomer-Polyurethan-Verstärkungsfilm und eine Elastomer-Hydrokolloidklebeschicht, welche zwischen der absorbierenden Schicht und der Verstärkungsschicht zum Ankleben der absorbierenden Schicht an der Verstärkungsschicht angeordnet ist, wobei die absorbierende Schicht ein schmelzgebundenes Blatt aus Elastomermaterial umfasst, welches an einem absorbierenden Nadelfilz vernadelt ist, welcher gelierende Fasern umfasst, wobei vorzugsweise die Fasern Alginat, Carboxymethylcellulose (CMC) und Partikel aus Silbercarbonat umfassen, welche gemeinsam gesponnen werden, um Fasern zu bilden, welche jeweils das Alginat, die Carboxymethylcellulose (CMC) und die Silbercarbonatpartikel umfassen, wobei optionsweise

die Elastomer-Verstärkungsschicht porös (beispielsweise perforiert) ist, und die absorbierende Schicht in dem Verband dergestalt ausgerichtet ist, dass das elastische, schmelzgebundene Elastomerblatt distal zur Wunde im Gebrauch in Bezug auf den absorbierenden Nadelfilz positioniert ist, und der absorbierende Nadelfilz so konfiguriert ist, dass er in der Lage ist, in direktem Kontakt mit der Wunde im Gebrauch zu sein, und wobei optionsweise

der Wundverband eine absorbierende Schicht, einen porösen Elastomer-Polyurethan-Verstärkungsfilm umfasst; und eine Elastomer-Hydrokolloidklebeschicht, welche zwischen der absorbierenden Schicht und dem Verstärkungsfilm angeordnet ist, um die absorbierende Schicht an den Verstärkungsfilm anzukleben, wobei die absorbierende Schicht aus einer schmelzgebundenen Bahn aus Elastomer-Polyurethan besteht, welche an einem absorbierenden Nadelfilz, welcher gelierende Fasern umfasst, vernadelt ist, wobei die Fasern Alginat, Carboxymethylcellulose (CMC) und Partikel aus Silbercarbonat umfassen, welche gemeinsam gesponnen werden, um Fasern zu bilden, welche jeweils das Alginat, die Carboxymethylcellulose (CMC) und die Silbercarbonatpartikel umfassen, wobei die absorbierende Schicht in dem Verband dergestalt ausgerichtet ist, dass die elastische, schmelzgebundene Elastomerbahn im Gebrauch distal zur Wunde in Bezug auf den absorbierenden Nadelfilz positioniert ist, welcher im Gebrauch relativ näher an der Wunde positioniert ist, und der Verband so konfiguriert ist, dass der absorbierende Nadelfilz in direktem Kontakt mit der Wunde im Gebrauch verwendbar ist, wobei der Verband ferner eine weitere an einer proximalen Fläche des Verbandes angeordnete Elastomer-Hydrokolloid-Klebeschicht umfasst, zum Ankleben der proximalen Fläche des Verbandes an die Haut eines Benutzers, wobei die weitere Elastomer-Hydrokolloidschicht mit Fenstern versehen ist, um die absorbierende Schicht in die Lage zu versetzen, direkt mit der Wunde im Gebrauch in Kontakt gebracht zu werden.

## Revendications

1. Pansement résilient comprenant :

une couche absorbante comprenant une feuille résiliente non-tissée de matériau élastomère collée à un matériau absorbant non-tissé ;
une couche de support élastomère ; et
une couche adhésive élastomère située entre la couche absorbante et la couche de support pour faire adhérer la couche absorbante à la couche de support,
dans lequel la feuille résiliente non-tissée et le matériau absorbant non-tissé sont collés par aiguilletage.

2. Pansement résilient selon la revendication 1, dans lequel :

a) la feuille résiliente non-tissée comprend une toile polymère réalisée par extrusion soufflage, facultativement dans lequel la toile polymère réalisée par extrusion soufflage comprend des fibres de polyuréthanes ; et/ou
b) le matériau non-tissé absorbant est un tissu non-tissé absorbant, de préférence un feutre aiguilleté, facultativement dans lequel le tissu comprend des fibres gélifiantes, et facultativement en outre dans lequel :

i) les fibres gélifiantes comprennent un alginate, une carboxyméthylcellulose (CMC), une carboxyméthylviscose, de la gélatine, de la pectine, de l'hydroxyéthyl-cellulose, de l'hydroxypropylcellulose, de la méthylcellulose, de la cellulose sulfonée (SC), de la viscose sulfonée, du carboxyméthylchitosane, de l'alcool de polyvinyle, ou toute combinaison de ceux-ci ; et/ou
ii) les fibres gélifiantes comprennent un alginate et une carboxyméthylcellulose (CMC), de préférence dans lequel l'alginate et la carboxyméthylcellulose (CMC) sont co-filés pour former des fibres qui comprennent chacune l'alginate et la carboxyméthylcellulose (CMC).

3. Pansement résilient selon l'une quelconque des revendications précédentes, dans lequel le matériau absorbant non-tissé comprend en outre un agent anti-microbien et/ou un agent anti-biofilm ; facultativement dans lequel l'agent anti-microbien et/ou l'agent anti-biofilm est sélectionné dans le groupe constitué d'argent, de composés d'argent, de composés d'iode, de monoguanides, de biguanides, de tensioactifs cationiques, de biocides, d'agents de dispersion, et d'une combinaison de ceux-ci, et facultativement en outre dans lequel l'agent anti-microbien et/ou l'agent anti-biofilm est un composé d'argent, de préférence un carbonate d'argent.

4. Pansement résilient selon l'une quelconque des revendications précédentes, dans lequel :

la couche de support élastomère est un film de support en polyuréthane, de préférence un film de support en polyuréthane perforé ; et/ou

le pansement résilient comprend en outre une autre couche adhésive élastomère située sur une surface proximale du pansement pour faire adhérer la surface proximale du pansement à la peau d'un utilisateur, de préférence dans lequel l'autre couche adhésive élastomère est ajourée ; et/ou

la couche adhésive élastomère et/ou l'autre couche adhésive élastomère comprend indépendamment un hydrocolloïde, facultativement dans lequel l'hydrocolloïde comprend de la carboxyméthylcellulose de sodium, des polysaccharides et de la pectine ; et/ou

le matériau absorbant non-tissé est dans une configuration plissée.

5. Procédé de fabrication d'un pansement résilient selon l'une quelconque des revendications précédentes, comprenant :

(a) le collage de la feuille résiliente non-tissée au matériau absorbant non-tissé pour former la couche absorbante, dans lequel le collage comprend un aiguilletage ; et
(b) l'adhésion de la couche absorbante à la couche de support élastomère en utilisant la couche adhésive élastomère.

6. Procédé de fabrication d'un article absorbant résilient pour un pansement comprenant :

l'expansion d'une feuille résiliente non-tissée de matériau élastomère d'un état préexpansé à un état expansé ; et
le collage d'un matériau absorbant non-tissé à la feuille résiliente non-tissée expansée dans lequel le collage comprend l'aiguilletage ; et
le fait de permettre à la feuille résiliente non-tissée de revenir de l'état expansé à l'état préexpansé pour fournir l'article absorbant résilient.

7. Procédé selon la revendication 5, dans lequel l'étape de collage (a) comprend :

l'expansion de la feuille résiliente non-tissée de matériau élastomère d'un état préexpansé à un état expansé ; et
le collage du matériau absorbant non-tissé à la feuille résiliente non-tissée dans lequel le collage comprend un aiguilletage ; et
le fait de permettre à la feuille résiliente non-tissée de revenir de l'état expansé à l'état préexpansé pour fournir la couche absorbante.

8. Procédé selon la revendication 6, dans lequel le procédé comprend en outre l'adhésion de l'article absorbant à une couche de support élastomère en utilisant une couche adhésive élastomère.

9. Procédé selon l'une quelconque des revendications 5 à 8,

dans lequel à la suite du collage de la feuille résiliente non-tissée au matériau absorbant non-tissé, le matériau absorbant non-tissé adopte une configuration plissée ; et/ou
dans lequel la feuille résiliente non-tissée comprend une toile polymère réalisée par extrusion soufflage, facultativement dans lequel la toile polymère non-tissée comprend des fibres de polyuréthane.

10. Procédé selon l'une quelconque des revendications 5 à 9, dans lequel le matériau non-tissé absorbant est un tissu absorbant non-tissé, de préférence dans lequel le tissu comprend des fibres gélifiantes, et facultativement en outre dans lequel les fibres gélifiantes comprennent un alginate, une carboxyméthylcellulose (CMC), une carboxyméthylviscose, de la gélatine, de la pectine, de l'hydroxyéthylcellulose, de l'hydroxypropylcellulose, de la méthylcellulose, de la cellulose sulfonée (SC), de la viscose sulfonée, du carboxyméthylchitosane, de l'alcool de polyvinyle, ou toute combinaison de ceux-ci ; facultativement dans lequel les fibres gélifiantes comprennent un alginate et une carboxyméthylcellulose (CMC), de préférence dans lequel l'alginate et la carboxyméthylcellulose (CMC) sont co-filés pour former des fibres qui comprennent chacune l'alginate et la carboxyméthylcellulose (CMC).

11. Procédé selon l'une quelconque des revendications 5 à 11, dans lequel le matériau absorbant non-tissé comprend en outre un agent anti-microbien et/ou agent anti-biofilm ; facultativement dans lequel l'agent anti-microbien et/ou l'agent anti-biofilm est sélectionné dans le groupe constitué d'argent, de composés d'argent, de composés d'iode, de monoguanides, de biguanides, de tensioactifs cationiques, de biocides, d'agents de dispersion et d'une combinaison de ceux-ci.

**12.** Procédé selon la revendication 11, dans lequel l'agent anti-microbien et/ou l'agent anti-biofilm est un composé d'argent, de préférence un carbonate d'argent ; facultativement dans lequel les fibres gélifiantes comprennent un alginate, une carboxyméthylcellulose (CMC) et un composé d'argent, de préférence dans lequel l'alginate, la carboxyméthylcellulose (CMC) et le composé d'argent sont co-filés ensemble pour former des fibres qui comprennent, chacune, l'alginate, la carboxyméthylcellulose (CMC) et le composé d'argent.

**13.** Procédé selon l'une quelconque des revendications 5 à 12, dans lequel en outre la couche de support élastomère est un film de support de polyuréthane, de préférence un film de polyuréthane perforé ; et/ou le procédé comprend en outre l'application d'une couche adhésive élastomère ajourée à une surface proximale de la couche absorbante ou de l'article.

**14.** Article absorbant résilient comprenant une aiguille en matériau non-tissé absorbant collée à une feuille résiliente de matériau élastomère de sorte que l'article absorbant adopte une configuration plissée quand l'article absorbant n'est pas sous tension et soit capable d'adopter une configuration sensiblement aplatie quand l'article absorbant est sous tension ; facultativement, dans lequel la feuille résiliente de matériau élastomère est une feuille résiliente non-tissée en matériau élastomère.

**15.** Pansement selon la revendication 1, comprenant une couche absorbante, un film de support en polyuréthane élastomère et une couche adhésive d'hydrocolloïde élastomère située entre la couche absorbante et la couche de support pour faire adhérer la couche absorbante à la couche de support, la couche absorbante comprenant une feuille collée par fusion de l'aiguille en matériau élastomère collée à un feutre aiguilleté absorbant comprenant des fibres gélifiantes, de préférence dans lequel les fibres comprennent un alginate, une carboxyméthylcellulose (CMC) et des particules de carbonate d'argent co-filés ensemble pour former des fibres qui comprennent chacune l'alginate, la carboxyméthylcellulose (CMC) et des particules de carbonate d'argent, facultativement dans lequel :
la couche de support élastomère est poreuse (par exemple perforée) et la couche absorbante est orientée dans le pansement de sorte que la feuille élastomère résiliente collée par fusion soit positionnée de manière distale par rapport à la blessure pendant l'utilisation relativement au feutre aiguilleté absorbant et de sorte que le feutre aiguilleté absorbant soit configuré de façon à pouvoir être en contact direct avec la blessure pendant l'utilisation, et facultativement dans lequel :
le pansement comprend une couche absorbante, un film de support en polyuréthane poreux élastomère ; et une couche adhésive hydrocolloïde élastomère située entre la couche absorbante et le film de support pour faire adhérer la couche absorbante au film de support, la couche absorbante étant constituée d'une toile collée par fusion d'un polyuréthane élastomère qui est aiguilleté à un feutre aiguilleté absorbant comprenant des fibres gélifiantes, dans lequel les fibres comprennent un alginate, une carboxyméthylcellulose (CMC) et des particules de carbonate d'argent co-filés ensemble pour former des fibres qui comprennent, chacune, l'alginate, la carboxyméthylcellulose (CMC) et les particules de carbonate d'argent, dans lequel la couche absorbante est orientée dans le pansement de sorte que la toile élastomère résiliente réalisée par extrusion soufflage soit positionnée de manière distale par rapport à la blessure lors de l'utilisation relativement au feutre aiguilleté absorbant qui est positionné de manière relativement plus proximale à la blessure lors de l'utilisation et le pansement est configuré de sorte que le feutre aiguilleté absorbant soit utilisable pour être en contact direct avec la blessure pendant l'utilisation, le pansement comprenant en outre une autre couche adhésive hydrocolloïde élastomère située sur une surface proximale du pansement pour faire adhérer la surface proximale du pansement à la peau d'un utilisateur, dans lequel l'autre couche hydrocolloïde élastomère est ajourée de façon à permettre à la couche absorbante d'être en contact direct avec la blessure pendant l'utilisation.

Figure 1

Figure 2a

Figure 2b

Figure 3

Figure 4

Figure 5

Figure 6

Figure 7

Figure 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010035017 A1